(19) Europäisches Patentamt
European Patent Office
Office européen des brevets

(11) **EP 2 430 979 B1**

(12) **EUROPEAN PATENT SPECIFICATION**

(45) Date of publication and mention
of the grant of the patent:
**16.12.2015   Bulletin 2015/51**

(51) Int Cl.:
*A61B 8/12* *(2006.01)*        *A61B 6/03* *(2006.01)*
*A61B 1/267* *(2006.01)*       *A61B 8/14* *(2006.01)*
*A61B 1/00* *(2006.01)*        *A61B 8/08* *(2006.01)*

(21) Application number: **10827691.6**

(22) Date of filing: **26.10.2010**

(86) International application number:
**PCT/JP2010/068973**

(87) International publication number:
**WO 2011/062035 (26.05.2011 Gazette 2011/21)**

(54) **BIOPSY SUPPORT SYSTEM**

BIOPSIEUNTERSTÜTZENDES SYSTEM

SYSTÈME DE SUPPORT DE BIOPSIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.11.2009   JP 2009262314**

(43) Date of publication of application:
**21.03.2012   Bulletin 2012/12**

(73) Proprietor: **Olympus Corporation
Shibuya-ku
Tokyo (JP)**

(72) Inventor: **ONISHI, Junichi
Shibuya-ku
Tokyo 151-0072 (JP)**

(74) Representative: **Gunzelmann, Rainer
Wuesthoff & Wuesthoff
Patentanwälte PartG mbB
Schweigerstraße 2
81541 München (DE)**

(56) References cited:
**EP-A1- 1 181 893          WO-A1-2004/042546
WO-A1-2007/055032          WO-A2-2008/111070
JP-A- 2000 116 655          JP-A- 2001 198 125
JP-A- 2004 097 696          JP-A- 2004 097 696
JP-B- 4 022 192          US-A1- 2005 215 854**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to a biopsy support system that supports, when an ultrasound probe is inserted into the bronchus or the like, treatment of biopsy from a target tissue such as a tumor using an ultrasound tomographic image.

Background Art

In recent years, ultrasound probes as well as endoscopes are widely used for various therapies and treatments. Ultrasound probes are used, for example, to extract the peripheral bronchus as an ultrasound tomographic image.

When a tissue such as a tumor exists in an ultrasound tomographic image, it may be necessary to perform a biopsy on the tissue and make a diagnosis.

On the other hand, as a first related art example, Japanese Patent Application Laid-Open Publication No.2004-499 discloses an ultrasound medical system that outputs information on the position of a target tissue such as a tumor acquired by transmitting or receiving ultrasound using an ultrasound transducer as appropriate information so as to be used with an X-ray radiator or the like provided aside from the ultrasound transducer.

In such a related art example, when position information of the tumor relative to a reference position is generated, a treatment through X-ray radiation into the tumor is performed using an X-ray radiator.

However, in a case where a tumor was detected, it is sometimes difficult to determine whether or not the tumor is to be treated through X-ray radiation. With respect to a target tissue such as the tumor, diagnosis of which thus has not been determined yet, biopsy (tissue extraction) is widely performed.

Note that, as a second related art example, Japanese Patent No.4022192 discloses an insertion support system provided with virtual image generating means for generating a virtual image of a body cavity path in a subject based on image data in a three-dimensional region of the subject, route start point setting means for setting a start point of an insertion route to the body cavity path, interested region setting means for setting an interested region in the body of the subject and route end point extracting means for extracting an end point of the insertion route of an endoscope into the body cavity path based on the interested region.

According to the second related art example, an operator can set an endoscope and the like in the vicinity of the interested region according to the insertion route. However, after that, it is difficult for the operator to perform tissue extraction, since there is no disclosure regarding actual tissue extraction from a target tissue such as a tumor using a biopsy device.

The operator can thus set a distal end portion of the endoscope, for example, in the vicinity of the target tissue according to the second related art example. However, information for performing tissue extraction on the target tissue by the distal end portion of the endoscope is insufficient.

Document WO 2008/111070 A1 discloses an apparatus for use with an imaging device that acquires a pre-procedure image of a portion of a subject's body disposed at a pre-procedure position. An input unit receives from a user an indication of two or more lines within the image that correspond to respective branches of a multi-branch luminal structure. A probe moves within the portion along two or more lines in the structure that respectively correspond to the two or more lines within the image. A location sensor coupled to the probe senses location coordinates along the two or more lines, while the portion is at a current position. A control unit registers the image of the portion with the portion's current position by registering the two or more lines within the image, with the location coordinates along the two or more lines.

Document WO 2004/042546 A1 discloses an imaging apparatus comprising a first device, for obtaining a first image, by a first modality, selected from the group consisting of SPECT, PET, CT, an extracorporeal gamma scan, an extracorporeal beta scan, x-rays, an intracorporeal gamma scan, an intracorporeal beta scan, an intravascular gamma scan, an intravascular beta scan, and a combination thereof, and a second modality, selected from the group consisting of a three-dimensional ultrasound, an MRI operative by an internal magnetic field, an extracorporeal ultrasound, an extracorporeal MRI operative by an external magnetic field, an intracorporeal ultrasound, an intracorporeal MRI operative by an external magnetic field, an intravascular ultrasound, and a combination thereof. The apparatus further includes a computerized system configured to construct an attenuation map, for the first image, based on the second, structural image. The computerized system is configured to display an attenuation corrected first image as well as a superposition of the attenuation-corrected first image and the second, structural image.

Document US 2005/215854 A1 discloses a medical procedure support system including an endoscope for obtaining images of an internal part of the body cavity of a subject, an endoscopic image creating unit for creating an endoscopic image obtained by the endoscope, an image reading unit for reading a virtual image relating to the subject and a reference image relating to the virtual image, a superimposition commanding unit for commanding to superimpose the reference image on at least one of the virtual image and the endoscopic image, and a combined image creating unit for performing the superimposition of the reference image data commanded by the superimposition commanding unit and creating a combined image thereof.

Therefore, a biopsy support system is desired, which includes a function for supporting biopsy by displaying information on a biopsy range for facilitating tissue extraction by a biopsy device, such as a range from the position of the distal end portion of the endoscope to the location where the target tissue exists.

The present invention has been achieved in view of the above-described points, and an object of the present invention is to provide a biopsy support system which is capable of displaying a biopsy range for facilitating tissue extraction on a target tissue such as a tumor, or information on the biopsy range.

Disclosure of Invention

Means for Solving the Problem

A first aspect of a biopsy support system of the present invention includes the features of claim 1. A second aspect of a biopsy support system of the present invention includes the features of claim 2,

Brief Description of the Drawings

Fig. 1 is a diagram illustrating an overall configuration of a biopsy support system of Embodiment 1 of the present invention;
Fig. 2 is a diagram illustrating a configuration of a distal end side of an ultrasound probe;
Fig. 3 is a flowchart illustrating an example of operation procedure for performing a biopsy according to Embodiment 1;
Fig. 4 is a flowchart illustrating processing of generating and displaying-a virtual image of the bronchus;
Fig. 5 is a block diagram illustrating a detailed configuration of the insertion support apparatus or the like in Fig. 1;
Fig. 6 is a diagram illustrating a state in which the distal end portion of an endoscope is inserted in the peripheral side of the bronchus;
Fig. 7 is a diagram illustrating the ultrasound probe inserted into the channel of the endoscope whose distal end protrudes toward the tumor side;
Fig. 8 is a flowchart illustrating an example of processing procedure for detecting the position of a tumor and calculating an appropriate range for a biopsy;
Fig. 9 is a diagram illustrating how the distal end portion of the ultrasound probe is moved to detect the range in which the tumor exists using an ultrasound tomographic image;
Fig. 10 is a diagram illustrating an example of calculation of an appropriate range;
Fig. 11 is a diagram illustrating examples of images when no tumor is detected on the ultrasound tomographic image and when a tumor is detected;
Fig. 12 is a diagram illustrating a display example of virtual shape image of a virtual image of the bronchus displayed with information of an appropriate range or the like superimposed thereon;
Fig. 13 is a diagram illustrating an overall configuration of a biopsy support system according to Embodiment 2 of the present invention;
Fig. 14 is a flowchart illustrating an example of processing procedure for performing a biopsy according to Embodiment 2;
Fig. 15 is a diagram illustrating how the ultrasound probe is moved to generate ultrasound tomographic image data;
Fig. 16 is a flowchart illustrating an example of processing procedure for calculating an appropriate range along a direction perpendicular to a running direction of the bronchus in a modification example;
Fig. 17 is a diagram illustrating ultrasound tomographic image data acquired by the ultrasound transducer;
Fig. 18 is a diagram illustrating the position of the center of gravity of the tumor and an appropriate range calculated using the ultrasound tomographic image data in Fig. 17; and
Fig. 19 is a diagram illustrating a display example of a virtual shape image of the virtual image of the bronchus and a situation in which a tissue is extracted from the tumor using a biopsy needle protruding from the channel of the endoscope.

Best Mode for Carrying Out the Invention

Hereinafter, embodiments of the present invention will be described with reference to the accompanying drawings.

(Embodiment 1)

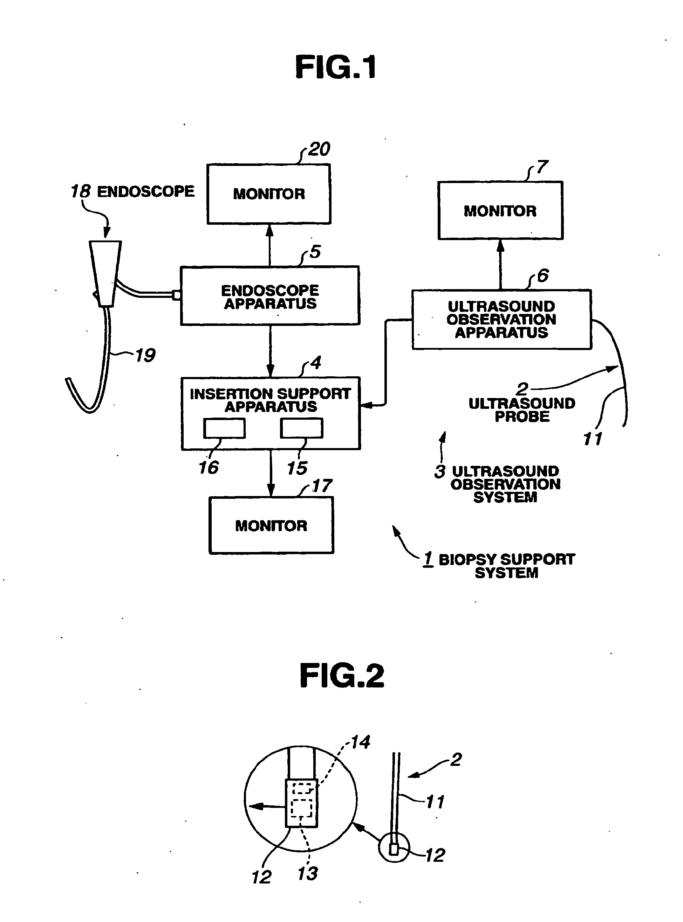

As shown in Fig. 1, a biopsy support system 1 of Embodiment 1 of the present invention is mainly configured by including an ultrasound observation system 3 provided with an ultrasound probe 2 inserted into, for example, the bronchus as a tubular body cavity path which is an insertion target, an insertion support apparatus 4 used together with the ultrasound observation system 3 and an endoscope apparatus 5.

The ultrasound observation system 3 includes the ultrasound probe 2, an ultrasound observation apparatus 6 to which the ultrasound probe 2 is connected and provided with an ultrasound image generating section that generates an ultrasound tomographic image and a monitor 7 that displays an ultrasound tomographic image generated by the ultrasound observation apparatus 6.

Furthermore, the ultrasound probe 2 has an elongated probe body 11. Fig. 2 shows an enlarged view of the distal end of the ultrasound probe 2. A probe distal end portion 12 as the distal end portion of the ultrasound probe 2 is provided with an ultrasound transducer 13 and a position sensor 14 arranged to detect the position of the probe distal end portion 12 or the ultrasound transducer 13.

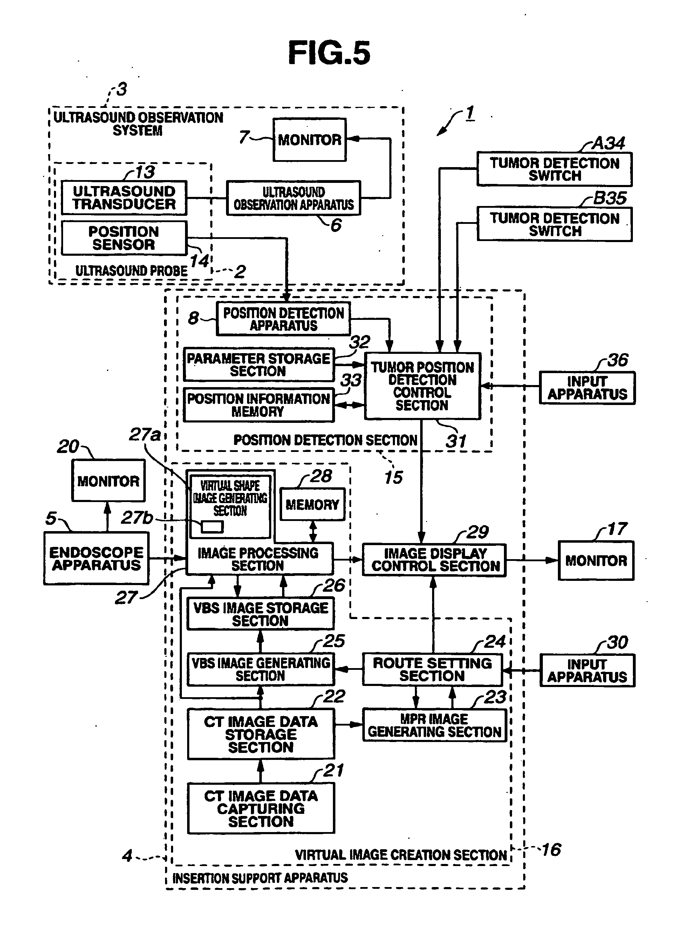

A position detection apparatus 8 (see Fig. 5) actually detects the three-dimensional position of the position sensor 14. The three-dimensional position can be approximated as a three-dimensional position of the ultrasound transducer 13 or probe distal end portion 12.

Since the distance between the position sensor 14 and the ultrasound transducer 13 is known, the three-dimensional position of the ultrasound transducer 13 can be detected accurately from the three-dimensional position of the position sensor 14 in consideration of this distance.

A method using magnetism is widely used as the position detection method. The position sensor 14 made up of a coil detects a fluctuating magnetic field emitted from an antenna (not shown) connected to the position detection apparatus 8 and the position detection apparatus 8 detects a current. The position detection apparatus 8 can estimate the position of the position sensor 14 from the fluctuating magnetic field outputted from the antenna and the current value of the position sensor 14. A magnetic position detection apparatus using a coil has been described as an example, but the position sensor 14 and the position detection apparatus 8 are not limited to this configuration.

The position sensor 14 is arranged in the probe distal end portion 12 together with the ultrasound transducer 13.

As will be described in detail in Fig. 5, the insertion support apparatus 4 includes a position detection section 15 provided with the position detection apparatus 8 that detects the three-dimensional position of the position sensor 14 using a tomographic image of the tumor as a target tissue detected on the ultrasound tomographic image to detect a boundary position or positions of both ends of a range in which the tumor exists in a predetermined direction (running direction of the body cavity path) based on the information of the three-dimensional position thereof. The position detection section 15 may also be defined to include a function of detecting a region in which the tumor exists in the direction orthogonal to the running direction of the body cavity path.

Furthermore, the insertion support apparatus 4 includes a virtual image creation unit 16 that generates a virtual image such as a virtual endoscope image (hereinafter referred to as "VBS image") of the bronchus as the body cavity path and a virtual three-dimensional shape image (virtual shape image) of the bronchus based on CT image data so as to be able to smoothly perform a processing procedure for performing a biopsy of the tumor as a target tissue. The VBS image as the virtual endoscope image of the bronchus is an endoscope image of the bronchus virtually estimated by the endoscope placed in the bronchus.

The insertion support apparatus 4 has a function of combining the endoscope image (real image) as a moving image obtained by the endoscope apparatus 5 and the VBS image and displaying the combined image on a monitor 17 and assists the operator in inserting an endoscope 18 of the endoscope apparatus 5 into the bronchus.

Furthermore, the endoscope apparatus 5 is configured by including the endoscope 18, a light source device that supplies illuminating light to the endoscope 18 and a camera control unit (abbreviated as "CCU") that performs signal processing on an image pickup signal from the endoscope 18.

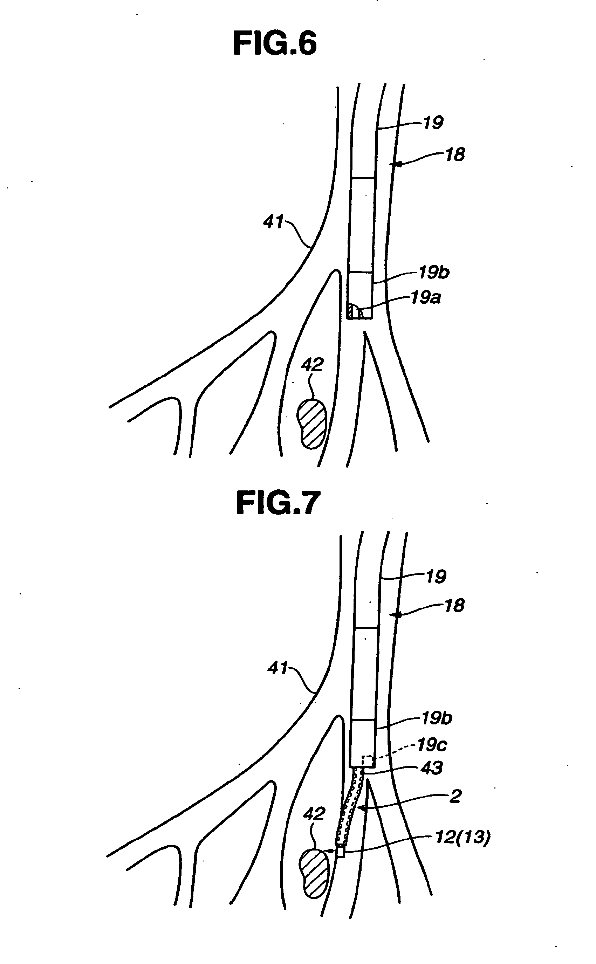

The endoscope 18 incorporates image pickup means 19c (see Fig. 7) in a distal end portion 19b of an elongated endoscope insertion portion 19 and an image pickup signal captured by the image pickup means 19c is subjected to signal processing by the CCU and an image signal corresponding to the real image is generated. The image signal is outputted to a monitor 20 and the real image is displayed on the monitor 20.

The endoscope 18 is provided with a channel 19a in the longitudinal direction of the endoscope insertion portion 19 (see Fig. 6). The ultrasound probe 2 and a biopsy device such as a biopsy needle as a biopsy treatment instrument for performing a biopsy can be inserted into the channel 19a. Furthermore, a bendable bending portion is provided adjacent to the proximal end portion of the distal end portion 19b of the endoscope insertion portion 19 and the operator can insert the distal end side of the endoscope into the curved body cavity path (inside the bronchus in a specific example) through an operation of bending the bending portion.

The ultrasound probe 2 inserted into the channel 19a of the endoscope 18 in the present embodiment has a sufficiently small diameter and has a small size. Furthermore, the ultrasound transducer 13 incorporated in the ultrasound probe 2 used in the present embodiment is of a radial scan type that transmits/receives ultrasound in a circumferential direction around the axis of the ultrasound probe 2 in the longitudinal direction. The ultrasound transducer 13 is not limited to the radial scan type, but may be of a sector-scanning type that scans partially in the circumferential direction.

When the operator inserts the endoscope 18 into the bronchus (as a subject) of the patient, an image captured by image pickup means is displayed on the monitor 20 as a real image. The operator can insert the endoscope 18 close to the position of the tumor which is the biopsy target on the peripheral side of the bronchus while observing the bronchus with the real image displayed on the monitor 20 with reference to the VBS image displayed on the monitor 17.

In this case, referencing also the virtual shape image of the bronchus generated by the virtual image creation unit 16, which will be described later, will make it easier to insert the endoscope into the peripheral side of the bronchus. Fig. 5 is a configuration block of the present system. This will be described first.

The virtual image creation unit 16 making up the insertion support apparatus 4 includes a CT image data capturing section 21 that captures three-dimensional image data generated by a publicly known CT apparatus (not shown) that picks up an X-ray tomographic image of the patient via a portable storage medium such as a DVD (Digital Versatile Disk) apparatus.

Furthermore, the virtual image creation unit 16 includes a CT image data storage section 22 that stores the CT image data captured by the CT image data capturing section 21 and an MPR image generating section 23 that generates an MPR image (multi-planar reconstruction image) based on the CT image data stored in the CT image data storage section 22.

Furthermore, the virtual image creation unit 16 also includes an image processing unit 27 that receives an image pickup signal from the endoscope apparatus 5 as an input signal, generates a real image and combines the real image with a VBS image. The image processing unit 27 is connected to a memory 28 that temporarily stores the image data and the like when performing image processing.

Furthermore, the image processing unit 27 also incorporates a virtual shape image generating section 27a that generates a virtual shape image of the bronchus from the CT image data stored in the CT image data storage section 22. The virtual shape image generating section 27a stores the generated virtual shape image data of the bronchus in an image data storage section 27b provided therein. The image data storage section 27b forms a virtual shape image storage section that stores the virtual shape image. The virtual shape image generating section 27a may also be provided outside the image processing unit 27.

Furthermore, the insertion support apparatus 4 is provided with an image display control section 29 that controls the monitor 17 so as to display a route setting screen generated by the route setting section 24 and an insertion support image generated by the image processing unit 27. Furthermore, an input apparatus 30 is provided which is made up of a keyboard and a pointing device or the like that inputs setting information to the route setting section 24.

The CT image data storage section 22 and a VBS image storage section 26 that stores a VBS image may be made up of one hard disk or the MPR image generating section 23, the route setting section 24, a VBS image generating section 25 that generates a VBS image and the image processing unit 27 may also be made up of one calculation processing circuit.

Furthermore, although the CT image data capturing section 21 has been described as one that captures CT image data via a portable storage medium such as a DVD, when the CT apparatus or an in-hospital server that stores CT image data is connected to an in-hospital LAN, the CT image data capturing section 21 may be made up of an interface circuit connectable to the in-hospital LAN so as to capture the CT image data via the in-hospital LAN.

Furthermore, the position detection section 15 is provided with a tumor position detection control section 31 that detects a boundary position or the like of the tumor as a target tissue using the three-dimensional position information detected by the position detection apparatus 8, a parameter storage section 32 that stores appropriate range parameters used to determine an appropriate range as a range appropriate for a biopsy and a position information memory 33 that stores the detected position information.

The target tissue is not limited to (the tissue of) the tumor but may be the (tissue of) the diseased part or the like to be subjected to a biopsy by the operator.

The tumor position detection control section 31 is connected to a tumor detection switch A34 that receives as input of a tumor detection signal, information indicating that one end of the tumor located along a predetermined direction in which the probe distal end portion 12 is moved is detected and a tumor detection switch B35 that receives no tumor (detection) signal as input. The operator performs input operations on the tumor detection switch A34 and the tumor detection switch B35. As in the case of an embodiment which will be described later, the configuration may be adapted such that the apparatus making up the biopsy support system generates a tumor detection signal and a no tumor signal.

Since an ultrasound tomographic image acquired using the ultrasound transducer 13 in the distal end portion of the ultrasound probe 2 making up the ultrasound observation system 3 is displayed on the monitor 7, the operator can check from the ultrasound tomographic image whether or not the tumor is ready to be detected (displayed).

The operator can then check the presence/absence of a tumor outside the tubular body cavity (body cavity) of the bronchus which is not optically observable on the ultrasound tomographic image while inserting/moving the ultrasound probe 2 along the running direction of the bronchus, for example, on the peripheral side.

While moving the ultrasound probe 2, the operator operates, when detecting one end (e.g., top end) of the tumor on an ultrasound tomograph, the tumor detection switch A34 to input a tumor detection signal to the tumor position detection control section 31. The tumor position detection control section 31 then stores the three-dimensional position of the distal end portion of the ultrasound probe 2, that is, the probe distal end portion 12 outputted by the position detection apparatus 8 in the position information memory 33.

Furthermore, the tumor detection switch B35 is provided to which the operator inputs information indicating the other end of the tumor (that is, portion where the tumor will no more be detected from the other end) as a no tumor signal to the tumor position detection control section 31.

Here, an example has been described where when the end of the tumor is detected, a tumor detection signal and no tumor signal are inputted using two means; tumor detection switch A34 and tumor detection switch B35. It may also be possible to use only the tumor detection switch A34 and change the duration of time that the switch is held down to thereby recognize a tumor detection signal or no tumor signal.

When the no tumor signal is inputted, the tumor position detection control section 31 stores the information of the three-dimensional position of the probe distal end portion 12 detected by the position detection apparatus 8 in the position information memory 33.

Thus, as will be described later, the range in which the tumor is located in at least a predetermined direction (running direction of the bronchus into which the probe distal end portion 12 is inserted) or the positions of both ends are enabled to be detected.

Furthermore, the tumor position detection control section 31 performs processing of calculating an appropriate range suitable for a biopsy using a biopsy treatment instrument as a biopsy range in the tumor using the information of the three-dimensional position of the probe distal end portion 12. The tumor position detection control section 31 then outputs the information of the three-dimensional position of the probe distal end portion 12 and the information of the appropriate range of the tumor to the image display control section 29.

Furthermore, the information of the three-dimensional position of the probe distal end portion 12, position information of appropriate range or the biopsy target position are stored in the position information memory 33 making up storage means for storing position information.

The image display control section 29 associates the information of the three-dimensional position of the probe distal end portion 12 with a virtual image to make it easier for the operator to recognize the region of the bronchus with respect to which the ultrasound image is acquired. That is, the image display control section 29 associates the sensor coordinate system calculated using the position sensor 14 with a CT coordinate system which constitutes the virtual image. To make both coordinate systems associated with each other, parameters necessary for a conversion are acquired or set from a plurality of known positions.

For example, by setting the patient in a predetermined position and defining the running direction of the bronchus, the operator gives an instruction for setting the probe distal end portion 12 at one known point near the inlet of the bronchus and associating the sensor coordinate system with the CT coordinate system. The operator further gives an instruction for sequentially inserting the probe distal end portion 12 into the bronchus by a plurality of known insertion lengths to associate the sensor coordinate system with the CT coordinate system. Thus, the present system 1 allows parameters necessary for a conversion from one coordinate system to the other to be acquired.

Upon receiving the information of the three-dimensional position of the probe distal end portion 12 or the like from the tumor position detection control section 31, the image display control section 29 displays the position of the probe distal end portion 12 or the like superimposed on the position associated therewith on the virtual image of the bronchus displayed on the monitor 17. That is, the image display control section 29 displays (or performs control so as to display) the position information of the probe distal end portion 12 and information such as the biopsy range superimposed on the virtual image of the bronchus.

Furthermore, an input apparatus 36 such as a keyboard is provided as data input means to allow the operator to input data specifying the biopsy target position such as substantially the center position of the region in which the tumor is located to the tumor position detection control section 31.

Furthermore, from the input apparatus 36, the operator can instruct the tumor position detection control section 31 to record the information of the three-dimensional position detected by the position detection apparatus 8 in the position information memory 33.

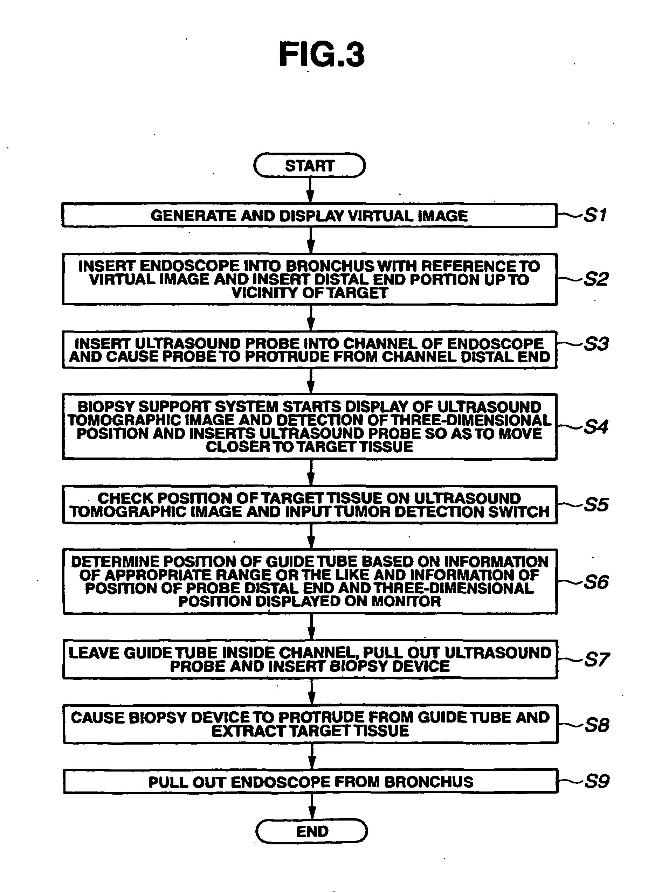

Fig. 3 shows a typical example of the procedure for performing a biopsy using the present insertion support system 1. Hereinafter, the operation and configuration of the present insertion support system 1 will be described in detail according to this procedure.

First, in first step S1 in Fig. 3, the operator acquires three-dimensional CT image data of the patient, generates a virtual image of the patient using the insertion support apparatus 4 and causes a virtual image to be displayed on the monitor 17.

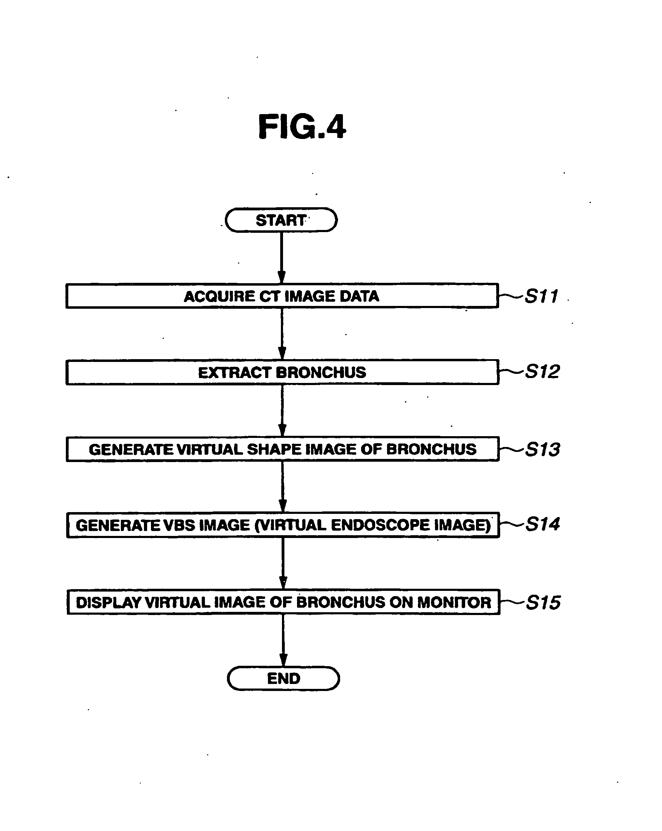

The processing is performed by the virtual image creation unit 16 and Fig. 4 shows details thereof.

In first step S 11 in Fig. 4, the operator acquires three-dimensional CT image data of the patient by CT scan before a treatment of extracting a tissue.

The three-dimensional CT image data is captured into the CT image data storage section 22 in the insertion support apparatus 4 via the CT image data capturing section 21 in the virtual image creation unit 16 incorporated in the insertion support apparatus 4. The three-dimensional CT image data can be easily moved with a DVD, for example.

In next step S12, the image processing unit 27 performs bronchus extraction processing through the processing of extracting, for example, the body cavity portion where air exists. Furthermore, in step S 13, the image processing unit 27 generates a virtual shape image of the extracted bronchus. The image processing unit 27 stores the image data of the generated virtual shape image of the bronchus in the image data storage section of the memory 28 or the like and sends the image data to the image display control section 29.

The virtual image creation unit 16 includes a route setting section 24 that generates a route setting screen having the MPR image (multi-planar reconstruction image) generated by the MPR image generating section 23 and sets a route to the bronchus of the endoscope 18. Furthermore, the virtual image creation unit 16 also includes a VBS image generating section 25 that generates a VBS image according to the CT image data stored in the CT image data storage section 22 and the route set by the route setting section 24.

The CT image data in the CT image data storage section 22 is outputted to the MPR image generation section 23. While viewing the MPR image generated here, the operator sets a route using the input means 30. The route setting section 24 determines the route using the route setting information and the MPR image data. The VBS image generating section 25 generates a VBS image from the CT image data based on the route information (step S 14).

The VBS image generated by the VBS image generating section 25 is stored in the VBS image storage section 26.

In next step S15, the image display control section 29 causes the monitor 17 to display the virtual image of the bronchus. In this case, the monitor 17 also displays the VBS image generated by the VBS image generating section 25 synthesized with the virtual image. Therefore, the monitor 17 displays a virtual image made up of the VBS image of the bronchus and the virtual shape image.

The virtual image can be thereby generated and displayed according to the flow shown in Fig. 4.

Next, as shown in step S2 in Fig. 3, the operator inserts the endoscope 18 up to the peripheral side of the target bronchus with reference to the virtual image of the bronchus. Fig. 6 shows the situation in which the distal end portion 19b of the endoscope 18 is inserted close to the target region near the tumor 42 of the peripheral side of the bronchus 41.

After that, as shown in step S3, the operator inserts the ultrasound probe 2 into the channel 19a of the endoscope 18 and causes the distal end side of the ultrasound probe 2 to protrude from an opening at the distal end of the channel 19a.

Fig. 7 shows the distal end side of the ultrasound probe 2 that protrudes from the distal end opening of the channel 19a of the endoscope 18 up to the vicinity of a position where the tumor 42 becomes observable.

The broken line shows the ultrasound probe 2 which is inserted into the channel 19a of the endoscope 18 via a guide tube 43. The guide tube 43 is set such that the distal end opening thereof is located, for example, in the vicinity of the proximal end of the probe distal end portion 12 of the ultrasound probe 2.

The guide tube 43 is moved inside the channel 19a together with the ultrasound probe 2. The guide tube 43 can be used to position the distal end side when performing a treatment of extracting a tissue using the biopsy device as a treatment instrument for conducting a biopsy treatment, which will be described below.

Furthermore, as shown in step S4, the biopsy support system 1 starts the display of an ultrasound tomographic image by the ultrasound observation system 3 and the operation of detecting the three-dimensional position using the position detection apparatus 8 of the insertion support apparatus 4. The position detection apparatus 8 then detects the three-dimensional position of the probe distal end portion 12 in which the position sensor 14 is provided. The operator grasps the position of the target tissue from the ultrasound image and the three-dimensional position information and inserts the ultrasound probe 2 so as to move closer to the target tissue.

Here, suppose the position of the position detection sensor 14 is $P_1$, the position of the ultrasound transducer 13 is $P_2$ and the distance between the position detection sensor 14 and the ultrasound transducer 13 is 1. When the distance 1 is small, for example, l<1 mm, approximation $P_1 \approx P_2$ is possible. The following equation is used to calculate the accurate position $P_2$ of the ultrasound transducer 13. Assuming that the ultrasound transducer 13 is located in the direction of the direction vector of the position detection sensor 14, that is, the ultrasound transducer 13 and the position detection sensor 14 are located on the same straight line, the position of $P_2$ is given by $P_1(x_1, y_1, z_1)$, $P_2(x_2, y_2, z_2)$, direction vector $n=(x_3, y_3, z_3)$ and distance 1 as follows

$$\mathbf{P_2 = P_1 + ln}$$

That is,

[Formula 1]

$$\begin{pmatrix} x_2 \\ y_2 \\ z_2 \end{pmatrix} = \begin{pmatrix} x_1 \\ y_1 \\ z_1 \end{pmatrix} + l \begin{pmatrix} x_3 \\ y_3 \\ z_3 \end{pmatrix} \cdot = \begin{pmatrix} x_1 + l\,x_3 \\ y_1 + l\,y_3 \\ z_1 + l\,z_3 \end{pmatrix}$$

Next, as shown in step S5, the operator checks the position of the tumor 42 as the target tissue while observing the ultrasound tomographic image by the ultrasound transducer 13 in the probe distal end portion 12 caused to protrude from the opening at the distal end of the channel 19a. In this case, the position detection section 15 derives an appropriate range of biopsy based on the inputs of the tumor detection switches A34 and B35.

Furthermore, as shown in step S6, the tumor position detection control section 31 sends information of the appropriate range of biopsy or the like calculated in step S5 to the image display control section 29. Furthermore, the image display control section 29 causes the appropriate range of biopsy (or information on the appropriate range) to be displayed superimposed on the position corresponding to the appropriate range on the virtual image generated by the image processing unit 27.

Furthermore, the tumor position detection control section 31 sends the information of the three-dimensional position of the probe distal end portion 12 to the image display control section 29. The image display control section 29 performs image processing of superimposing (information on) the appropriate range or the like and the three-dimensional position of the probe distal end portion 12 of the ultrasound probe 2 on the virtual image of the bronchus 41 and displays the superimposed image on the monitor 17. The operator then determines the position of the guide tube based on the information displayed on the monitor 17.

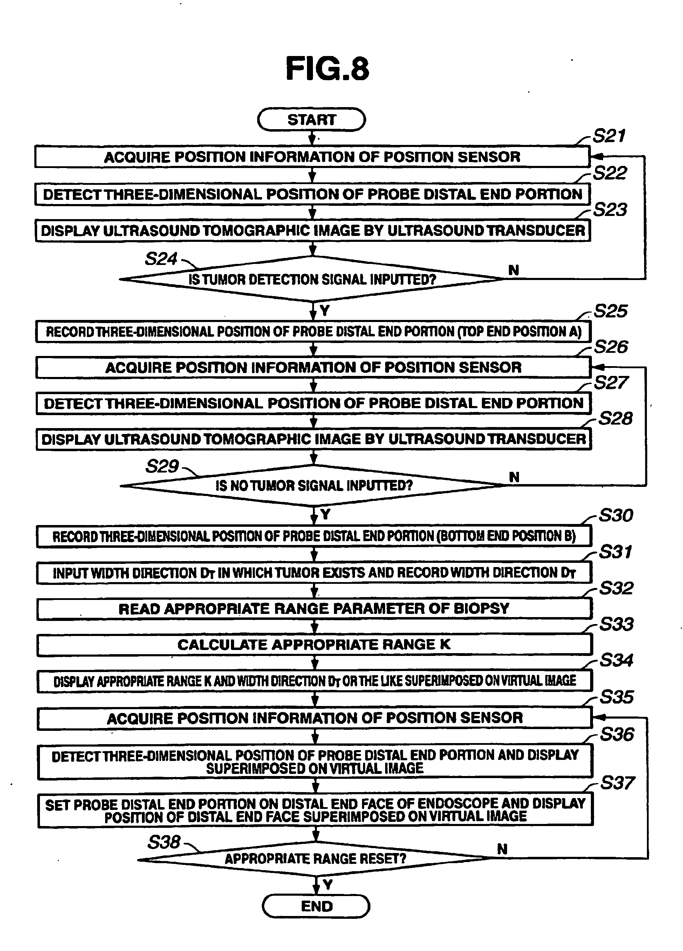

Thus, the information of the three-dimensional position is converted to the CT coordinate system used for the image processing of the CT image data and the image processing of generating the virtual image of the bronchus 41 and displayed superimposed on the virtual image of the bronchus 41. Performing such a display makes it easier to perform a treatment when a tissue is extracted from the tumor 42. Fig. 8 illustrates the processing flow including position detection of the tumor by the biopsy support system 1 in the procedure from steps S4 to S6 in Fig. 3.

Next, the procedure including position detection of the tumor 42 as the target tissue in step S5 in Fig. 3 will be described with reference to Fig. 8.

As described above, when the position detection apparatus 8 is set in an operating state, the position detection apparatus 8 acquires three-dimensional position information of the position sensor 14 as shown in step S21. As shown in step S22, the position detection apparatus 8 detects the three-dimensional position of the position sensor 14, in other words, the three-dimensional position of the probe distal end portion 12.

Furthermore, as shown in step S23, the ultrasound transducer 13 incorporated in the probe distal end portion 12 transmits/receives ultrasound and the ultrasound tomographic image generated by the ultrasound image generating section inside the ultrasound observation apparatus 6 is displayed on the monitor 7.

The operator moves the probe distal end portion 12 to the peripheral side thereof along the running direction of the bronchus 41 while observing the ultrasound tomographic image displayed on the monitor 7. The operator causes the probe distal end portion 12 to contact the inner wall surface of the bronchus 41 so as to obtain an ultrasound tomographic image. The probe distal end portion 12 may also be provided with a balloon that accommodates an ultrasound transmission medium so as to be able to contact with the inner wall surface of the bronchus 41 and thereby reduce transmission loss of ultrasound.

As shown in next step S24, the tumor position detection control section 31 is set in a state of waiting for input of the tumor detection switch A34 and repeats the processing from step S21 to step S24 until a tumor detection signal by the tumor detection switch A34 is inputted.

As shown in Fig. 7, when the ultrasound transducer 13 of the probe distal end portion 12 reaches a boundary position where the tumor 42 starts to be observed, it is possible to obtain an ultrasound tomographic image corresponding to the tumor 42 (which may also be simply referred to as "tomographic image") on the monitor 7.

After the positioning of the guide tube 43 ends in step S6 in Fig. 3 above, as shown in step S7, the operator pulls out the ultrasound probe 2 leaving the guide tube 43 in the channel 19a and inserts the biopsy device into the guide tube 43.

After that, as shown in step S8, the operator causes the biopsy device to protrude from the distal end of the positioned guide tube 43 and performs a biopsy, that is, extraction of a tissue of the tumor 42 as the target tissue. After the tissue extraction, as shown in step S9, the operator pulls out the endoscope 18 together with the biopsy device from the bronchus 41. The operator then finishes the treatment of tissue extraction of the target tumor 42.

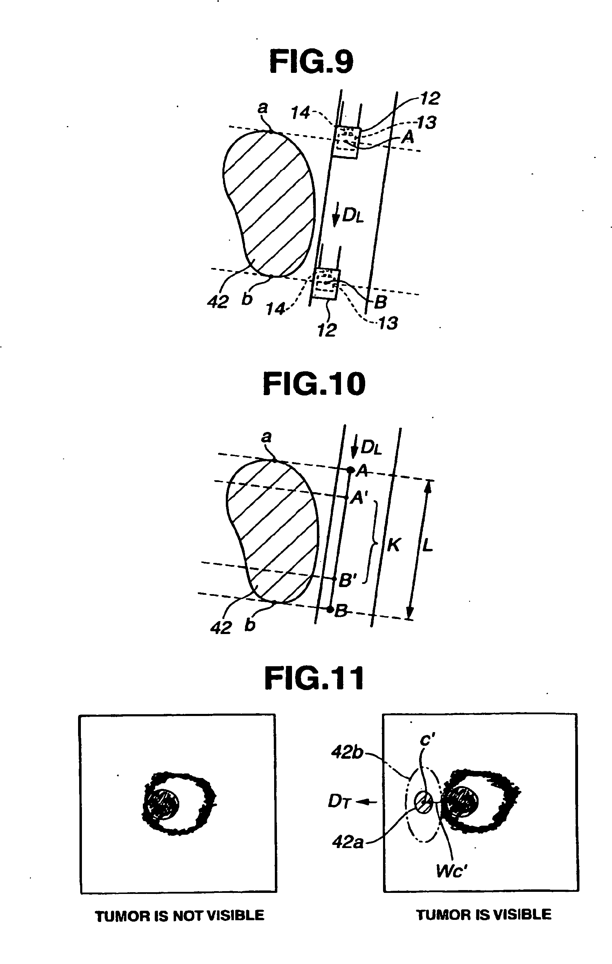

Fig. 9 is an enlarged view of the peripheral part of the tumor 42 in Fig. 7. As shown in Fig. 9, when (the ultrasound transducer 13 of) the probe distal end portion 12 moves in the running direction $D_L$ of the bronchus 41 and the probe distal end portion 12 reaches a position A (top end position A) in the running direction $D_L$ corresponding to a top end position a of the tumor 42, it is possible to observe a tomographic image of the tumor 42. Fig. 11 schematically illustrates the ultrasound tomographic image displayed on the monitor 7 in this case. Since the tumor 42 has an acoustic impedance different from that of a normal tissue, the tumor 42 can be identified according to the luminance level or the like on the ultrasound tomographic image.

From the state of the ultrasound tomographic image shown on the left side of Fig. 11, a tomographic image 42a of the tumor 42 is displayed as shown on the right side.

The ultrasound tomographic image in Fig. 11 corresponds to a case where a radial scan is performed, that is, the ultrasound transducer 13 sequentially radiates ultrasound in a circumferential direction perpendicular to the axis of the ultrasound probe 2 in a longitudinal direction. Furthermore, Fig. 11 shows a case where the direction perpendicular to the surface of the sheet is assumed to be the running direction $D_L$ and the small circle represents the transmission/reception position of ultrasound by the ultrasound transducer.

Upon judging that there is a tissue to be subjected to a biopsy while viewing the difference in the ultrasound tomographic image of the monitor 7 (the difference in the portion between a normal tissue and the tumor 42 as the tissue to be subjected to a biopsy), the operator operates the tumor detection switch A34 and inputs a tumor detection signal.

In step S25 in Fig. 8, the tumor position detection control section 31 stores the three-dimensional position of the probe distal end portion 12, namely, a three-dimensional top end position A, which is a top end position a of the tumor 42 projected in the running direction $D_L$, in the position information memory 33 as means for storing the position information.

The operator further continues the operation of moving the probe distal end portion 12 in the running direction $D_L$ of the bronchus 41 and looks for the other end of the tumor 42 while viewing the ultrasound tomographic image.

For this operation, the processing from step S26 to step S29 is performed. Step S26, step S27 and step S28 have the same processing as that in step S21, step S22 and step S23, respectively. Furthermore, in step S29, the tumor position detection control section 31 is set in a state of waiting for input of the tumor detection switch B35 and the processing in step S26 to step S29 is repeated until no tumor signal is inputted by the tumor detection switch B35.

When the probe distal end portion 12 crosses a bottom end position b of the tumor 42 in Fig. 9, the ultrasound tomographic image changes from the state on the right side in Fig. 11 to the state on the left side. When the state is changed to the state on the left side of Fig. 11, the operator operates the tumor detection switch B35 and inputs a no tumor signal.

In step S30 in Fig. 8, the tumor position detection control section 31 stores the three-dimensional position of the probe distal end portion 12, namely, a three-dimensional position B (bottom end position B) of the tumor 42 in the running direction $D_L$ corresponding to the bottom end position b in the position information memory 33.

When the operator inputs the direction in which the tumor 42 is located from the input apparatus 36 in step S31, for example, the tumor position detection control section 31 stores information on the direction in which the tumor 42 is located (in addition to the information of the three-dimensional positions of the top end position A and the bottom end position B) in the position information memory 33.

The direction in which the tumor 42 is located is not confirmed when there is only the information of the three-dimensional positions of the top end position A and the bottom end position B. To be more specific, it is not possible to determine in which direction of the circumferential direction around the axis of a line segment AB connecting the top end position A and the bottom end position B, the tumor 42 is located. Thus, when the probe distal end portion 12 is moved in the running direction $D_L$ of the bronchus 41, the operator inputs a direction in which the tomographic image 42a of the tumor 42 appears. For example, in the case of Fig. 11, the operator inputs a direction corresponding to 9 o'clock of a clock (as the width direction $D_T$ in which the tumor 42 appears).

The width direction $D_T$ in which the tumor 42 is located is thereby confirmed. The input operation of the width direction $D_T$ may also be performed between step S24 and step S29. The width direction $D_T$ may also be determined (inputted) through image processing as shown in Embodiment 2 which will be described later.

In step S31, in addition to the width direction $D_T$ or instead of the width direction $D_T$, the operator may also specify a substantially center position c' of the tumor 42 in the width direction $D_T$ (in a modification example of Embodiment 2 which will be described later, the width or range along the width direction $D_T$ in which the tumor 42 is located around the axis of the running direction $D_L$ may be detected or an appropriate range suitable for biopsy in the width direction $D_T$ is calculated).

For example, the two-dot dashed line in Fig. 11 indicates the tomographic image 42b of the tumor 42 obtained when the probe distal end portion 12 is set in the vicinity of the center position between the top end position A and the bottom end position B.

The operator specifies the center position c' along the width direction $D_T$ using a pointing device (not shown) provided in the ultrasound observation apparatus 6 (the ultrasound observation apparatus 6 may specify the center position c' through image processing in Embodiment 2 which will be described later). The ultrasound observation apparatus 6 calculates an actual distance Wc from a distance Wc' from the ultrasound transducer to the center position c' on the tomographic image 42b.

It is possible to convert the distance Wc' on the ultrasound tomographic image to the actual distance Wc using the values of parameters or the like used to display a tomographic image. The operator inputs the actual distance Wc from the input apparatus 36 (the ultrasound observation apparatus 6 may also specify the distance Wc in Embodiment 2 which will be described later). The information on the distance Wc is stored in the position information memory 33.

Furthermore, the information of the three-dimensional position of the center position c of the tumor 42 may also be stored in the position information memory 33. The center position c may also be set as the target position when performing a biopsy.

The operator may also specify the center position c' along the width direction $D_T$ using the above described pointing device, the ultrasound observation apparatus 6 may automatically calculate the actual distance Wc and output the information of the distance Wc to the tumor position detection control section 31 and the tumor position detection control section 31 may store the information of the distance Wc in the position information memory 33. In that case, the position information of the center position c of the tumor 42 may also be stored in the position information memory 33.

In next step S32, the tumor position detection control section 31 reads appropriate range parameters of biopsy from the parameter storage section 32. Furthermore, in next step S33, the tumor position detection control section 31 calculates an appropriate range of biopsy suitable for appropriately conducting a biopsy from the three-dimensional position information of the top end position A and the bottom end position B stored in the position information memory 33 and appropriate range parameters.

How to calculate the appropriate range K along the running direction $D_L$ of the tumor 42 will be described with reference to Fig. 10. Fig. 10 is basically the same figure as Fig. 9 and is a diagram illustrating how the appropriate range K is calculated from the top end position A and the bottom end position B using the ultrasound transducer 13 in Fig. 9.

As shown in Fig. 10, the length of a line segment AB between the top end position A and the bottom end position B that correspond to the top end position a and the bottom end position b where the tumor 42 is located along the running direction $D_L$ of the bronchus 41 becomes the length L of the tumor 42 along the direction $D_L$.

The appropriate range K may also be set as the length L of the tumor 42. However, the tumor 42 may have a shape different from the substantially ellipsoidal shape shown in Fig. 10, for example, a crescent shape of a small thickness. Therefore, when the appropriate range parameter is set so as to set or calculate the appropriate range K inside the detected both ends, it is possible to extract the tissue of the tumor 42 more reliably when performing a biopsy. For such a reason, the present embodiment sets the appropriate range K inside or on the center side of the detected line segment AB.

A top end position A' and a bottom end position B' of the appropriate range K are calculated, for example, using the following equations.

$$A'=(B-A)\times t/100+A$$

$$B'=B-(B-A)\times t/100$$

Here, t as an appropriate range parameter is a constant and can be set, for example, to 10 to on the order of tens. When the tumor is approximate to a sphere, t may be set to 10 or less.

In step S34 in Fig. 8, the tumor position detection control section 31 transfers the data of the calculated appropriate range K and width direction $D_T$ in which the tumor 42 is located or information of the center position c to the image display control section 29. The image display control section 29 displays information such as the width direction $D_T$ (or center position c) in which the appropriate range K and the tumor 42 are located as information of the biopsy range on the monitor 17 superimposed on the (virtual shape image in the) virtual image of the bronchus 41.

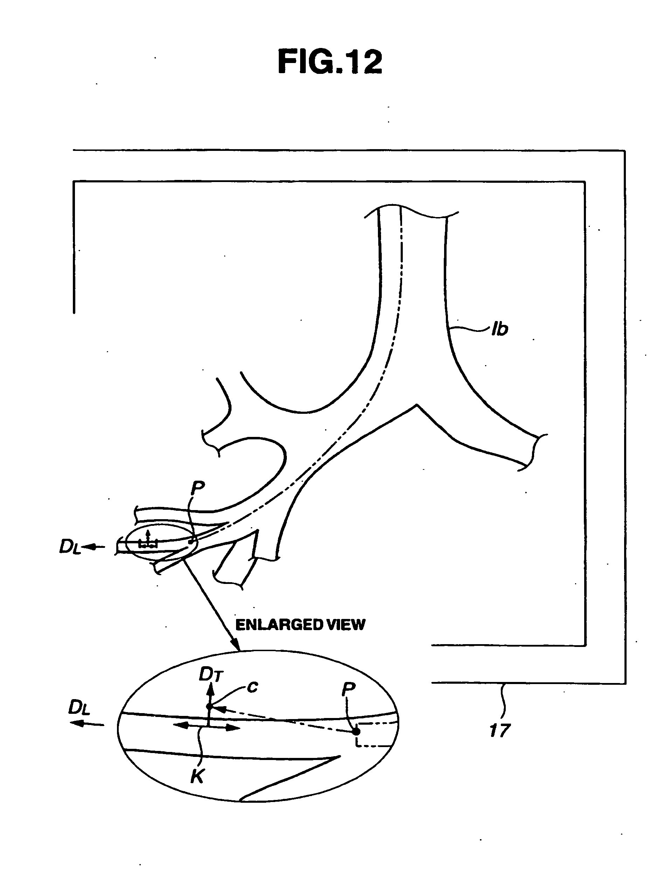

Fig. 12 illustrates one display example where the appropriate range K or the like is displayed on a virtual shape image Ib of the virtual image of the bronchus 41 displayed on the monitor 17. As shown in Fig. 12, the appropriate range K is displayed at a position corresponding to the appropriate range K on the virtual shape image Ib of the virtual image of the bronchus 41 and the width direction $D_T$ in which the tumor 42 is located is also displayed (in the direction corresponding to the width direction $D_T$).

Furthermore, the center position c of the tumor 42 in the width direction $D_T$ can also be displayed. The two-dot dashed line whose distal end corresponds to a reference position P in Fig. 12 indicates the insertion route of the endoscope 18.

Furthermore, as shown in step S35 next to step S34 in Fig. 8, the position detection apparatus 8 then acquires the position information of the position sensor 14. As shown in step S36, the position detection apparatus 8 detects the three-dimensional position of the probe distal end portion 12 and sends the information of the three-dimensional position to the image display control section 29. The three-dimensional position of the probe distal end portion 12 is displayed superimposed on the corresponding position on the virtual image Ib.

Therefore, for example, in step S37, the operator arranges the probe distal end portion 12 on the distal end face of the endoscope 18 (which corresponds to the distal end opening of the channel 19a). The three-dimensional position of the probe distal end portion 12 is acquired by the position detection apparatus 8.

The position corresponding to the three-dimensional position of the distal end face of the endoscope 18 (distal end opening of the channel 19a) is displayed, for example, as the reference position P on the virtual shape image Ib of the virtual image in Fig. 12. The position of the distal end opening of the guide tube 43 may also be displayed on the virtual shape image Ib of the virtual image as the reference position P.

The present embodiment displays the biopsy range as shown in Fig. 12 and information related thereto on the monitor 17, and can thereby support the operator so as to be able to smoothly position the biopsy device to extract a tissue from the tumor 42 by causing the biopsy device to protrude from the distal end face of the channel 19a of the endoscope 18.

In step S38 next to step S37 in Fig. 8, the tumor position detection control section 31 is set in a state of waiting for reset of the appropriate range K and if this operation is not performed, the processing in steps S35 to S38 is repeated.

For this reason, the operator may cause the operation to continue until, for example, the biopsy treatment of extracting a tissue from the tumor 42 ends. The operator then resets the appropriate range K at the end of tissue extraction and ends the position detection operation in Fig. 8.

After completing the acquisition of information such as the width direction $D_T$ in which the appropriate range K and the tumor 42 are located, and the center position c thereof to facilitate extraction of a tissue by the biopsy device using the ultrasound probe 2, and the processing of displaying the information superimposed on the virtual image, the operator pulls out the ultrasound probe 2 from the channel 19a.

That is, as described in step S7 in Fig. 3, the operator pulls out the ultrasound probe 2 from the channel 19a leaving the guide tube 43 and inserts the biopsy device.

The operator then causes the biopsy device to protrude from the distal end opening of the channel 19a of the endoscope 18 as shown in step S8 with reference to the virtual shape image Ib of the virtual image displayed in Fig. 12 and extracts a tissue from the tumor 42.

In this case, the operator causes the biopsy device to protrude in the direction of c shown by the one-dot dashed line using the position of the distal end opening of the channel 19a of the endoscope 18 as the reference position P in the enlarged view in Fig. 12, and can thereby extract a tissue from the tumor 42.

When a position sensor is provided at the distal end portion of the biopsy device, the position is detected by the position detection apparatus 8 and is also displayed on the virtual shape image Ib of the virtual image of the bronchus 41, and therefore the operator can more simply perform a treatment of biopsy. The position information of the biopsy device may also be stored in the position information memory 33.

Thus, according to Embodiment 1, a virtual image of the bronchus 41 is generated and information including the appropriate range K as a biopsy range calculated from the range existing along at least a predetermined direction (running direction of the body cavity path) of the tumor as a target tissue for the biopsy is displayed superimposed on the virtual image, and it is thereby possible to support the operator in facilitating the biopsy treatment.

(Embodiment 2)

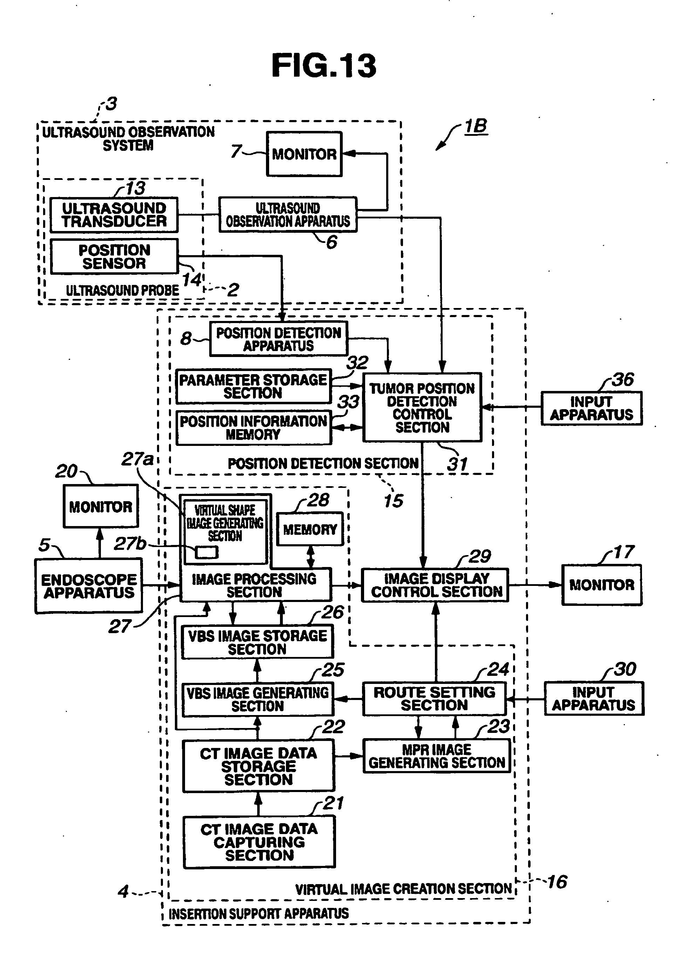

Next, Embodiment 2 of the present invention will be described. Fig. 13 illustrates a configuration of a biopsy support system 1B of Embodiment 2 of the present invention. The biopsy support system 1B has a configuration corresponding to, for example, the biopsy support system 1 in Fig. 5 without the tumor detection switch A34 and the tumor detection switch B35.

In the present embodiment, the biopsy support system 1B is configured to automatically perform, through image processing, the functions of the tumor detection switch A34 and the tumor detection switch B35 performed by the operator through input operation in Embodiment 1.

The image data of the ultrasound tomographic image generated by the ultrasound image generating section in the ultrasound observation apparatus 6 is inputted to the tumor position detection control section 31 and the tumor position detection control section 31 detects, through image processing, a time at which a tomographic image of the tumor 42 starts to be detected and a time at which the tomographic image ceases to be detected.

That is, the ultrasound probe 2 is moved along the running direction $D_L$ of the bronchus 41 and when (the ultrasound transducer 13 of) the probe distal end portion 12 crosses the top end position a of the tumor 42, the tumor position detection control section 31 generates a tumor detection signal (through an image change from the image on the left side in Fig. 11 to the image on the right side).

Likewise, after the tomographic image 42a of the tumor 42 becomes observable, when (the ultrasound transducer 13 of) the probe distal end portion 12 crosses the bottom end position b of the tumor 42, the tumor position detection control section 31 generates a no tumor signal (through an image change from the image on the right side in Fig. 11 to the image on the left side).

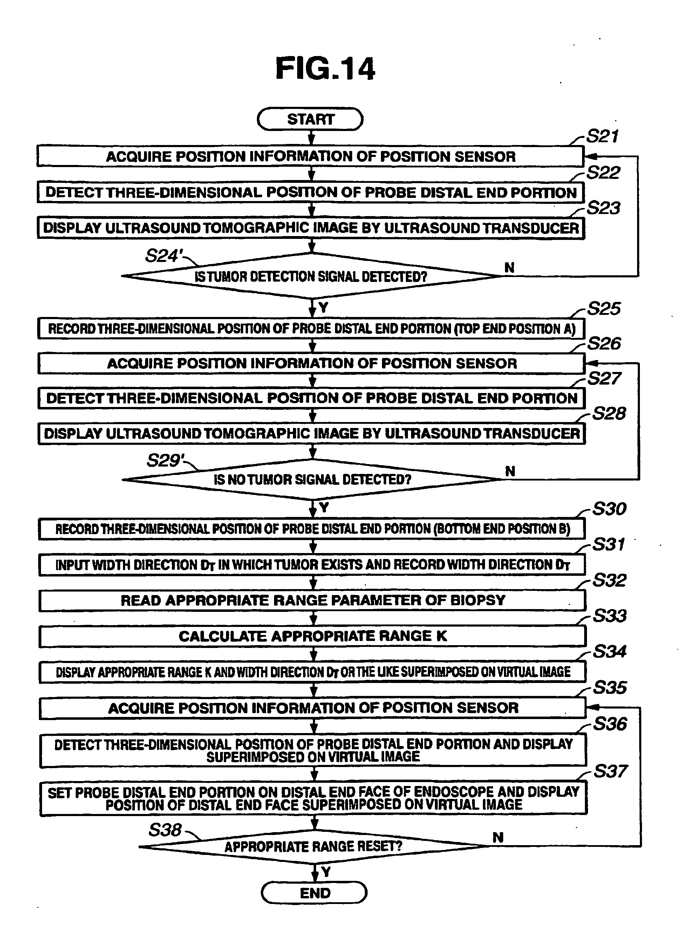

The rest of the configuration according to the present embodiment is the same as that of Embodiment 1. Fig. 14 illustrates a flowchart of operation of position detection according to the present embodiment.

The operation of position detection shown in Fig. 14 is only partially different from the processing in Fig. 8. To be more specific, step S24 and step S29 in Fig. 8 are changed to step S24' and step S29' as shown below.

That is, in step S24' next to step S23, the tumor position detection control section 31 decides on the ultrasound tomographic image whether or not the tomographic image of the tumor 42 starts to be detected, that is, a tumor detection signal is detected through the detection of a tomographic image of the tumor 42.

When this does not correspond to the detection of a tumor detection signal, the process returns to step S21. On the other hand, when the tomographic image of the tumor 42 is decided to start to be detected, that is, when a tumor detection signal is detected, the process moves to step S25.

Furthermore, in step S29' next to step S28, the tumor position detection control section 31 decides, on the ultrasound tomographic image, whether or not the tomographic image of the tumor 42 ceases to be detected, that is, a no tumor signal is detected through the detection of no tomographic image of the tumor 42.

When this does not correspond to the detection of no tumor signal, the process returns to step S26. On the other hand, when the detection of no tumor signal is decided, the process moves to step S30. The biopsy support system 1B inputs the width direction $D_T$ in which the tumor 42 is located in step S31 based on the tumor detection signal through image processing (instead of the operator doing so). The same applies to recording as well.

The rest of the operation is the same as that of Embodiment 1.

The present embodiment can reduce the burden on the operator more than Embodiment 1. The present embodiment has effects similar to those of Embodiment 1 in other aspects.

Next, a modification example of the present embodiment will be described.

In aforementioned Embodiment 1 and Embodiment 2, in addition to the appropriate range K of the running direction $D_L$, both end positions E and F along the width direction $D_T$ orthogonal to the direction $D_L$ may be detected as the biopsy range with respect to the tumor 42 from the position of the distal end face of the endoscope 18 and an appropriate range J of the width direction $D_T$ may be calculated.

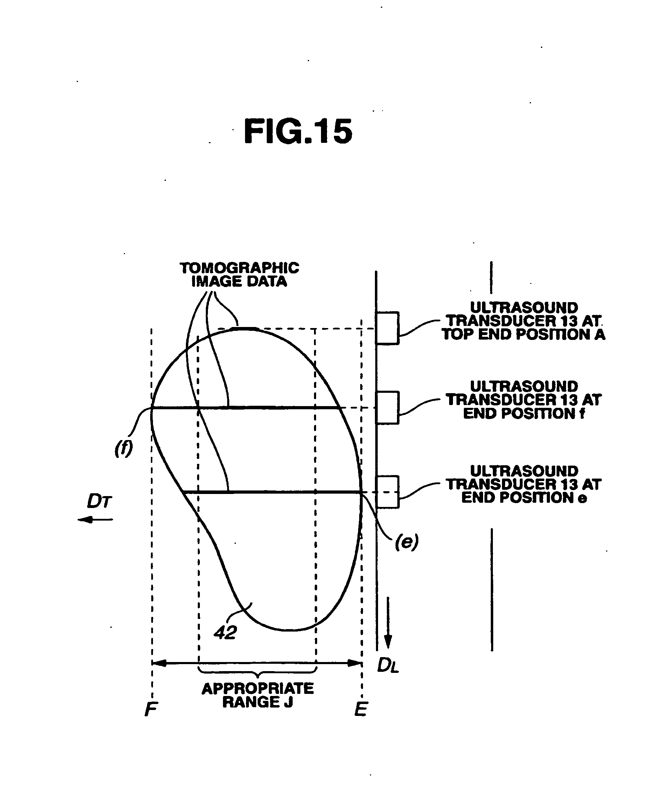

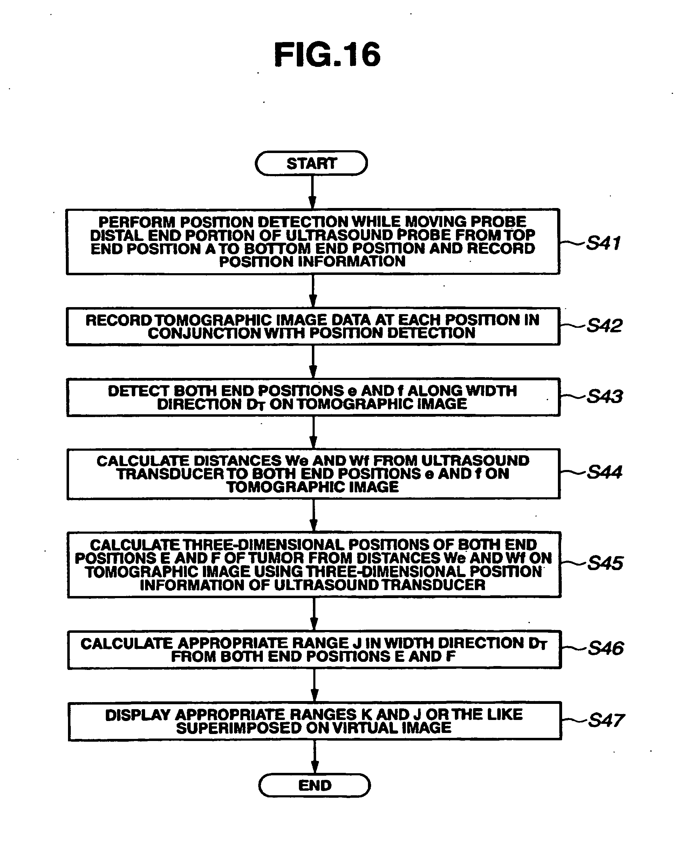

Fig. 15 shows a diagram illustrating this case. Fig. 16 illustrates a processing procedure in this case. As shown in step S41, the operator moves the probe distal end portion 12 of the ultrasound probe 2 in the running direction $D_L$ from the top end position A to the bottom end position B as in the case of Fig. 9. In this case, while moving, the tumor position detection control section 31 stores position detection information using the position sensor 14 by the position detection apparatus 8 in the position information memory 33.

In conjunction with this movement, as shown in step S42, the ultrasound image generating section in the ultrasound observation apparatus 6 sequentially records (ultrasound) tomographic image data obtained from the ultrasound transducer 13 in the memory in the ultrasound observation apparatus 6.

The position detection information by the position sensor 14 stored in the position information memory 33 is stored associated with the tomographic image data stored in the memory in the ultrasound observation apparatus 6.

Fig. 15 shows a representative position when the ultrasound probe 2 is moved in the running direction $D_L$ of the bronchus 41 and the tumor portion detected as tomographic image data (also referred to as "slice data") of the tumor 42 obtained in that case.

In next step S43, the ultrasound image generating section calculates, through image processing, an end position e at a minimum distance and an end position f at a maximum distance from the ultrasound transducer 13 along the width direction $D_T$ perpendicular to the running direction $D_L$ with respect to the volume data formed in the entire tomographic image data stored in the memory.

These end positions e and f are positions on the ultrasound tomographic image corresponding to the end position E at a minimum distance and the end position F at a maximum distance of the tumor 42 from the ultrasound transducer 13 and are shown with parentheses in Fig. 15.

Further, in next step S44, the image processing apparatus calculates distances We and Wf on the tomographic image from the ultrasound transducer 13 of the bronchus 41 in the running direction $D_L$ to the end positions e and f on the tomographic image. The ultrasound image generating section then outputs the calculated distances We and Wf on the tomographic image to the tumor position detection control section 31.

In next step S45, the tumor position detection control section 31 calculates the end positions E and F of the tumor 42 from the distances We and Wf on the tomographic image using the information of the three-dimensional position of the ultrasound transducer 13.

Furthermore, in next step S46, the tumor position detection control section 31 calculates the appropriate range J in the width direction $D_T$ perpendicular to the running direction $D_L$ using information on the end positions E and F as in the case of the appropriate range K along the running direction $D_L$.

In next step S47, the tumor position detection control section 31 outputs the calculated information of the appropriate range J in the width direction $D_T$ to the image display control section 29. The image display control section 29 causes the appropriate ranges K and J to be displayed superimposed on the virtual image of the bronchus 41 on the display plane of the monitor 17.

According to the present modification example, since the appropriate range J in the width direction $D_T$ can be displayed together with the appropriate range K in the running direction $D_L$, the operator can more easily perform a biopsy treatment using the biopsy device.

In Fig. 15, the appropriate range J in the width direction $D_T$ orthogonal to the running direction $D_L$ is calculated together with the appropriate range K in the running direction $D_L$.

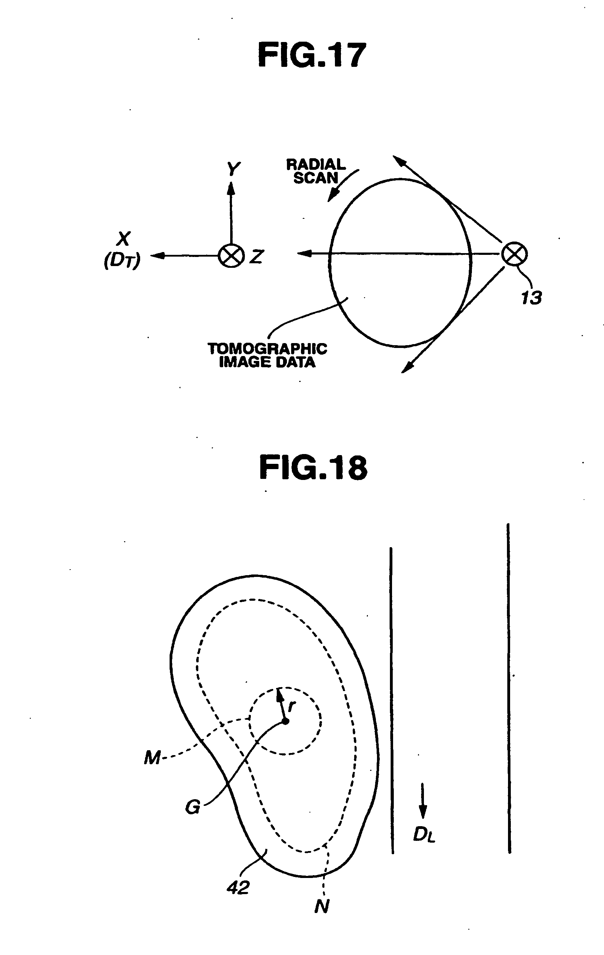

By contrast, a two-dimensional region of the appropriate range in the circumferential direction from the running direction $D_L$ may also be calculated together with the appropriate range K in the running direction $D_L$ as will be described below. The representative tomographic image data shown in Fig. 15 can be expressed on a plane perpendicular to the running direction $D_L$ of the bronchus 41 as shown in Fig. 17.

The ultrasound image generating section in the ultrasound observation apparatus 6 calculates, for example, a two-dimensional region in which the tomographic image data shown in Fig. 17 exists. In the example in Fig. 17, assuming the running direction $D_L$ as the Z coordinate, the two-dimensional region where the tomographic image data of the tumor 42 exists on the tomographic image plane (X and Y planes) perpendicular thereto is detected (where, the Z-axis is the coordinate axis of the running direction $D_L$ of the position detection apparatus 8).

The ultrasound image generating section performs detection of the two-dimensional region on the tomographic image data from the top end position A to the bottom end position B and sequentially outputs distribution information of the two-dimensional region in the tomographic image data to the tumor position detection control section 31.

The tumor position detection control section 31 converts the two-dimensional distribution of the tomographic image data on the tomographic image to a two-dimensional distribution on the position coordinate detection system using the position on the Z-axis coordinate system where the tomographic image data is acquired. The tumor position detection control section 31 performs the processing from the top end position A to the bottom end position B and thereby calculates a three-dimensional distribution region of the tumor 42 in a circumferential direction around the axis of the running direction $D_L$. The tumor position detection control section 31 calculates the three-dimensional distribution region of the tumor 42 and then calculates the center of gravity G thereof.

Furthermore, the tumor position detection control section 31 calculates the three-dimensional distribution region from the center of gravity G of the tumor 42 as shown in Fig. 18 and a spherical part within a predetermined distance r from the center of gravity G as an appropriate range M as shown in Fig. 18. The predetermined distance r is set so as to be within the three-dimensional distribution region of the tumor 42. Instead of the appropriate range M within the predetermined distance r from the center of gravity G, an appropriate range N may be calculated which is within a predetermined distance or inside at a predetermined rate from the peripheral end of the three-dimensional distribution region of the tumor 42.

The tumor position detection control section 31 outputs the three-dimensional position information of the calculated center of gravity G and appropriate range M or N to the image display control section 29. The image display control section 29 causes, for example, the appropriate range K and the center of gravity G, the appropriate range M or N, and reference position P of the distal end face of the endoscope 18 to be displayed superimposed on each other on the virtual image of the bronchus.

The image display control section 29 may also cause the appropriate range M or N to be additionally displayed as a second appropriate range together with the aforementioned appropriate range K or J or only the appropriate range M or N to be displayed instead of the appropriate range K or J.

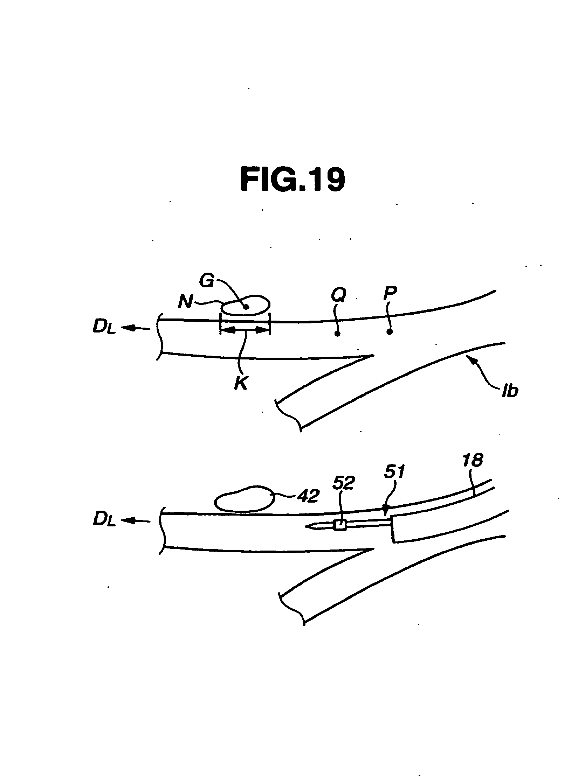

The upper side in Fig. 19 illustrates a display example of the virtual shape image Ib of the virtual image in this state. Furthermore, the under side in Fig. 19 illustrates a situation in which, for example, the biopsy needle 51 as the biopsy device is actually made to protrude from the distal end opening of the channel 19a of the endoscope 18 to extract a tissue from the tumor 42.

An example is shown here where a position sensor 52 is provided in the vicinity of the distal end portion of the biopsy needle 51. As described above, when the position sensor 52 is provided, the position Q thereof can be displayed superimposed on the virtual shape image Ib of the virtual image.

For example, in the case of Fig. 19, the direction PQ from the reference position P to the position Q is a direction deviated downward from the center of gravity G, and therefore the operator operates the bending portion of the endoscope 18 so that the direction PQ is directed to the center of gravity G (slightly curved upward on the surface of the sheet in Fig. 19). By causing the biopsy needle to protrude in the direction, the operator can extract a tissue from the tumor 42.

Thus, the operator can easily perform a treatment of extracting a tissue from the tumor 42 with reference to the virtual shape image Ib of the virtual image in the upper side of Fig. 19.

Furthermore, since the position detection means is incorporated in the biopsy device, there is no need for positioning by the guide tube 43, allowing the biopsy device to be guided according to the position information.

The aforementioned embodiments may be further modified. For example, in Fig. 9 or Fig. 10 of Embodiment 1, the top end positions A and B are detected as three-dimensional positions of the bronchus 41 of the ultrasound transducer 13 in the running direction $D_L$.

The top end position a and the bottom end position b of the tumor 42 may also be detected or calculated as modified detection positions. The top end positions a and b can be calculated with reference to the distance information from the ultrasound transducer 13 on the aforementioned tomographic image and the three-dimensional position information of the ultrasound transducer 13 when the tomographic image thereof is generated.

An appropriate range inside (center side of) the top end position a and the bottom end position b of the tumor 42 may be set as the biopsy range. Furthermore, the center positions of both ends may also be simultaneously calculated. The biopsy treatment may be effectively supported in this case, too.

Furthermore, the range in which the tumor 42 is located along the running direction $D_L$ or the like of the bronchus 41, for example, line segment AB as a guideline for performing a biopsy displayed superimposed on the virtual shape image Ib of the virtual image of the bronchus 41 also belongs to the present invention. That is, the range of both end positions in which the target tissue such as the tumor 42 is located in the running direction of the bronchus into which the probe distal end portion 12 is inserted and moved may be set as the biopsy range.

A case has been described in the aforementioned embodiments as an example where the virtual image creation unit 16 that generates a virtual image generates a virtual image made up of a VBS image as a virtual endoscope image of the bronchus as the body cavity path and a virtual shape image as a virtual three-dimensional shape image of the bronchus, but the present invention is also applicable to a configuration of the virtual shape image generating section in which the virtual image creation unit 16 generates only the virtual shape image of the body cavity path. Furthermore, the present invention is also applicable to a case where the virtual image creation unit 16 is a virtual endoscope image generating section that generates only a virtual endoscope image of the body cavity path.

Furthermore, although a case with the bronchus has been described as the body cavity path in the above described example, the present invention is not limited to this case but is also applicable to a case where the probe distal end portion 12 is inserted into the upper alimentary tract or lower alimentary tract and moved.

**Claims**

1. A biopsy support system (1B) comprising:

   a virtual shape image generating section (16) that generates a virtual shape image of a body cavity path (41) from image data in a three-dimensional region with respect to a subject;
   an endoscope (18) provided with an endoscope insertion portion (19) inserted into the body cavity path (41) and a channel (19a) that allows a biopsy treatment instrument (51) to pass therethrough;
   an ultrasound probe (2) inserted into the channel (19a) including an ultrasound transducer (13) and a position sensor (14) that detects a three-dimensional position provided at a distal end portion thereof; and
   an ultrasound image generating section (6) that generates an ultrasound tomographic image using the ultrasound transducer (13),
   **characterized in that** the biopsy support system (1B) further comprises
   a position detection section (15) that detects a three-dimensional position of the position sensor (14), when both end positions of a target tissue to be subjected to a biopsy are detected on the ultrasound tomographic image;
   a tumor position detection control section (31) that detects a boundary position of a tumor as the target tissue using the three-dimensional position of the position sensor (14) detected by the position detection section (15), wherein the tumor position detection control section (31) is connected to a tumor detection switch (A34) that receives as input of a tumor detection signal, information indicating that the distal end portion of the ultrasound probe (2) is moved along a predetermined direction and one end of the tumor is detected, and a tumor detection switch (B35) that receives no tumor detection signal as input, the tumor detection switch (A34) and the tumor detection switch (B35) being operated by an operator; and
   an image display control section (29) that displays a biopsy range in which the biopsy is performed on the target tissue using the biopsy treatment instrument (51), superimposed on a position corresponding to the biopsy range on the virtual shape image, based on the three-dimensional position corresponding to the both end positions detected by the position detection section (15), wherein
   the position detection section (15) detects three-dimensional positions of both end positions of the target tissue along the running direction ($D_L$) of the body cavity path (41), the both end positions being detected when the distal end portion of the ultrasound probe (2) is moved along the running direction ($D_L$), and detects the center or center of gravity of the target tissue detected along the width direction ($D_T$) orthogonal to the running direction ($D_L$) on the ultrasound tomographic image, and

the image display control section (29) further displays the range within both end positions of a target tissue along the running direction ($D_L$) as the biopsy range superimposed on a position corresponding to the biopsy range on the virtual shape image, and displays the center or center of gravity of the target tissue along the width direction ($D_T$) superimposed on the position corresponding to the center or center of gravity on the virtual shape image.

2. A biopsy support system (1B) comprising:

a virtual shape image generating section (16) that generates a virtual shape image of a body cavity path (41) from image data in a three-dimensional region with respect to a subject;

an endoscope (18) provided with an endoscope insertion portion (19) inserted into the body cavity path (41) and a channel (19a) that allows a biopsy treatment instrument (51) to pass therethrough;

an ultrasound probe (2) inserted into the channel (19a) including an ultrasound transducer (13) and a position sensor (14) that detects a three-dimensional position provided at a distal end portion thereof; and

an ultrasound image generating section (6) that generates an ultrasound tomographic image using the ultrasound transducer (13),

**characterized in that** the biopsy support system (1B) further comprises

a position detection section (15) that detects a three-dimensional position of the position sensor (14), when both end positions of a target tissue to be subjected to a biopsy are detected on the ultrasound tomographic image;

a tumor position detection control section (31) that detects a boundary position of a tumor as the target tissue using the three-dimensional position of the position sensor (14) detected by the position detection section (15), wherein the tumor position detection control section (31) generates the tumor detection signal when, through image processing, the ultrasound tomographic image of a tumor as the target tissue starts to be detected, and generates a no tumor detection signal when the ultrasound tomographic image ceases to be detected; and

an image display control section (29) that displays a biopsy range in which the biopsy is performed on the target tissue using the biopsy treatment instrument (51), superimposed on a position corresponding to the biopsy range on the virtual shape image, based on the three-dimensional position corresponding to the both end positions detected by the position detection section (15), wherein

the position detection section (15) detects three-dimensional positions of both end positions of the target tissue along the running direction ($D_L$) of the body cavity path (41), the both end positions being detected when the distal end portion of the ultrasound probe (2) is moved along the running direction ($D_L$), and detects the center or center of gravity of the target tissue detected along the width direction ($D_T$) orthogonal to the running direction ($D_L$) on the ultrasound tomographic image, and

the image display control section (29) further displays the range within both end positions of a target tissue along the running direction ($D_L$) as the biopsy range superimposed on a position corresponding to the biopsy range on the virtual shape image, and displays the center or center of gravity of the target tissue along the width direction ($D_T$) superimposed on the position corresponding to the center or center of gravity on the virtual shape image.

3. The biopsy support system (1B) according to claim 1 or 2, wherein the position detection section detects a three-dimensional position of a reference position set on a distal end face of the endoscope (18) or a periphery thereof, and the image display control section (29) displays the reference position superimposed on a position on the virtual shape image corresponding to the reference position.

4. The biopsy support system (1B) according to claim 1 or 2, wherein the image display control section (29) displays, assuming a range set inside the both end positions based on the both end positions as the biopsy range, the biopsy range superimposed on a position corresponding to the biopsy range on the virtual shape image.

5. The biopsy support system (1B) according to claim 1 or 2, wherein the position detection section detects a three-dimensional position of a center of gravity or a center position of the target tissue on the ultrasound tomographic image, and the image display control section (29) displays the center of gravity or center position superimposed on a position corresponding to the center of gravity or center position on the virtual shape image.

6. The biopsy support system (1B) according to claim 1 or 2, wherein the virtual shape image generating section (16) generates a virtual shape image of any one of the bronchus, the upper alimentary tract and the lower alimentary tract as a virtual shape image of the body cavity path (41).

7. The biopsy support system (1B) according to claims 1 or 2, wherein the biopsy treatment instrument (51) comprises a position sensor (52) near a distal end portion of the biopsy treatment instrument (51),

the position detection section detects a three-dimensional position of the position sensor (52) of the biopsy treatment instrument (51), and

the image display control section (29) displays the three-dimensional position of the position sensor (52) of the biopsy treatment instrument (51) superimposed on a corresponding position on the virtual shape image.

8. The biopsy support system (1B) according to claim 7, further comprising a storage section (33) that stores position information of both end positions of the target tissue detected by the position detection section (8) and position information of the position sensor (52) of the biopsy treatment instrument (51).


**Patentansprüche**

1. Biopsieunterstützungssystem (1B), das umfasst:

einen Abschnitt (16) zur Erzeugung eines Bilds einer virtuellen Form, der ein Bild einer virtuellen Form eines Körperhohlraumpfads (41) aus Bilddaten in einem dreidimensionalen Bereich bezüglich eines Subjekts erzeugt;
ein Endoskop (18), das mit einem in den Körperhohlraumpfad (41) eingeführten Endoskopeinführabschnitt (19) und einem Kanal (19a) versehen ist, durch den ein Biopsiebehandlungsinstrument (51) hindurchgeführt werden kann;
eine Ultraschallsonde (2), die in den Kanal (19a) eingeführt ist und einen Ultraschallwandler (13) und einen Positionssensor (14) umfasst, der eine an deren distalen Endabschnitt vorgesehene dreidimensionale Position erfasst;
einen Ultraschallbilderzeugungsabschnitt (6), der unter Verwendung des Ultraschallwandlers (13) ein tomographisches Ultraschallbild erzeugt,
**dadurch gekennzeichnet, dass** das Biopsieunterstützungssystem (1B) ferner umfasst
einen Positionserfassungsabschnitt (15), der eine dreidimensionale Position des Positionssensors (14) erfasst, wenn beide Endpositionen eines einer Biopsie zu unterziehenden Zielgewebes auf dem tomographisches Ultraschallbild erfasst werden;
einen Tumorpositionserfassungssteuerabschnitt (31), der unter Verwendung der von dem Positionserfassungsabschnitt (15) erfassten dreidimensionalen Position des Positionssensors (14) eine Grenzposition eines Tumors als das Zielgewebe erfasst, wobei der Tumorpositionserfassungssteuerabschnitt (31) mit einem Tumorerfassungsschalter (A34), der als Eingabe eines Tumorerfassungssignals eine Information empfängt, die anzeigt, dass der distale Endabschnitt der Ultraschallsonde (2) entlang einer vorbestimmten Richtung bewegt wird und ein Ende des Tumors erfasst wird, und einem Tumorerfassungsschalter (B35), der als Eingabe kein Tumorerfassungssignal empfängt, verbunden ist, wobei der Tumorerfassungsschalter (A34) und der Tumorerfassungsschalter (B35) durch einen Operateur betätigt werden; und
einen Bildanzeigesteuerabschnitt (29), der auf der Basis der dreidimensionalen Position, die den beiden durch den Positionserfassungsabschnitt (15) erfassten Endpositionen entspricht, einen Biopsiebereich, in dem unter Verwendung des Biopsiebehandlungsinstruments (51) die Biopsie an dem Zielgewebe durchgeführt wird, einer Position überlagert anzeigt, die dem Biopsiebereich auf dem Bild der virtuellen Form entspricht, wobei
der Positionserfassungsabschnitt (15) dreidimensionale Positionen von beiden Endpositionen des Zielgewebes entlang der Laufrichtung ($D_L$) des Körperhohlraumpfad (41) erfasst, wobei die beiden Endpositionen erfasst werden, wenn der distale Endabschnitt der Ultraschallsonde (2) entlang der Laufrichtung ($D_L$) bewegt wird, und den Mittelpunkt oder den Schwerpunkt des Zielgewebes, das entlang der Breitenrichtung ($D_L$) senkrecht zu der Laufrichtung ($D_L$) erfasst wird, auf dem tomographisches Ultraschallbild erfasst, und
der Bildanzeigesteuerabschnitt (29) ferner den Bereich zwischen beiden Endpositionen eines Zielgewebes entlang der Laufrichtung ($D_L$) als den Biopsiebereich einer Position überlagert anzeigt, die dem Biopsiebereich auf dem Bild der virtuellen Form entspricht, und den Mittelpunkt oder den Schwerpunkt des Zielgewebes entlang der Breitenrichtung ($D_L$) der Position überlagert anzeigt, die dem Mittelpunkt oder dem Schwerpunkt auf dem Bild der virtuellen Form entspricht.

2. Biopsieunterstützungssystem (1B), das umfasst:

einen Abschnitt (16) zur Erzeugung eines Bilds einer virtuellen Form, der ein Bild einer virtuellen Form eines Körperhohlraumpfads (41) aus Bilddaten in einem dreidimensionalen Bereich bezüglich eines Subjekts erzeugt;
ein Endoskop (18), das mit einem in den Körperhohlraumpfad (41) eingeführten Endoskopeinführabschnitt (19) und einem Kanal (19a) versehen ist, durch den ein Biopsiebehandlungsinstrument (51) hindurchgeführt werden kann;

eine Ultraschallsonde (2), die in den Kanal (19a) eingeführt ist und einen Ultraschallwandler (13) und einen Positionssensor (14) umfasst, der eine an deren distalen Endabschnitt vorgesehene dreidimensionale Position erfasst;

einen Ultraschallbilderzeugungsabschnitt (6), der unter Verwendung des Ultraschallwandlers (13) ein tomographisches Ultraschallbild erzeugt,

**dadurch gekennzeichnet, dass** das Biopsieunterstützungssystem (1B) ferner umfasst

einen Positionserfassungsabschnitt (15), der eine dreidimensionale Position des Positionssensors (14) erfasst, wenn beide Endpositionen eines einer Biopsie zu unterziehenden Zielgewebes auf dem tomographisches Ultraschallbild erfasst werden;

einen Tumorpositionserfassungssteuerabschnitt (31), der unter Verwendung der von dem Positionserfassungsabschnitt (15) erfassten dreidimensionalen Position des Positionssensors (14) eine Grenzposition eines Tumors als das Zielgewebe erfasst, wobei der Tumorpositionserfassungssteuerabschnitt (31) das Tumorerfassungssignal erzeugt, wenn, durch Bildverarbeitung begonnen wird, das tomographische Ultraschallbild eines Tumors als das Zielgewebe zu erfassen, und kein Tumorerfassungssignal erzeugt, wenn eine Erfassung des tomographische Ultraschallbild unterbleibt; und

einen Bildanzeigesteuerabschnitt (29), der auf der Basis der dreidimensionalen Position, die den beiden durch den Positionserfassungsabschnitt (15) erfassten Endpositionen entspricht, einen Biopsiebereich, in dem unter Verwendung des Biopsiebehandlungsinstruments (51) die Biopsie an dem Zielgewebe durchgeführt wird, einer Position überlagert anzeigt, die dem Biopsiebereich auf dem Bild der virtuellen Form entspricht, wobei der Positionserfassungsabschnitt (15) dreidimensionale Positionen von beiden Endpositionen des Zielgewebes entlang der Laufrichtung ($D_L$) des Körperhohlraumpfad (41) erfasst, wobei die beiden Endpositionen erfasst werden, wenn der distale Endabschnitt der Ultraschallsonde (2) entlang der Laufrichtung ($D_L$) bewegt wird, und den Mittelpunkt oder den Schwerpunkt des Zielgewebes, das entlang der Breitenrichtung ($D_L$) senkrecht zu der Laufrichtung ($D_L$) erfasst wird auf dem tomographisches Ultraschallbild erfasst, und der Bildanzeigesteuerabschnitt (29) ferner den Bereich zwischen beiden Endpositionen eines Zielgewebes entlang der Laufrichtung ($D_L$) als den Biopsiebereich einer Position überlagert anzeigt, die dem Biopsiebereich auf dem Bild der virtuellen Form entspricht, und den Mittelpunkt oder den Schwerpunkt des Zielgewebes entlang der Breitenrichtung ($D_L$) der Position überlagert anzeigt, die dem Mittelpunkt oder dem Schwerpunkt auf dem Bild der virtuellen Form entspricht.

3. Biopsieunterstützungssystem (1B) gemäß Anspruch 1 oder 2, bei dem der Positionserfassungsabschnitt eine dreidimensionale Position einer Referenzposition erfasst, die auf einer distalen Endfläche des Endoskops (18) oder deren Umfang festgelegt ist, und der Bildanzeigesteuerabschnitt (29) die Referenzposition einer Position auf dem Bild der virtuellen Form überlagert darstellt, die der Referenzposition entspricht.

4. Biopsieunterstützungssystem (1B) gemäß Anspruch 1 oder 2, bei dem der Bildanzeigesteuerabschnitt (29) unter der Annahme eines Bereichs, der auf der Basis der beiden Endpositionen innerhalb der beiden Endpositionen festgelegt ist, als den Biopsiebereich den Biopsiebereich einer Position überlagert darstellt, die dem Biopsiebereich auf dem Bild der virtuellen Form entspricht.

5. Biopsieunterstützungssystem (1B) gemäß Anspruch 1 oder 2, wobei der Positionserfassungsabschnitt eine dreidimensionale Position eines Schwerpunkts oder eine Mittelpunktsposition des Zielgewebes auf dem tomographische Ultraschallbild erfasst, und der Bildanzeigesteuerabschnitt (29) den Schwerpunkt oder die Mittelpunktsposition einer Position überlagert darstellt, die dem Schwerpunkt oder der Mittelpunktsposition auf dem Bild der virtuellen Form entspricht.

6. Biopsieunterstützungssystem (1B) gemäß Anspruch 1 oder 2, bei dem der Abschnitt (16) zur Erzeugung eines Bilds einer virtuellen Form ein Bild einer virtuellen Form der Bronchie, des oberen Verdauungstrakt oder des unteren Verdauungstrakt als ein Bild einer virtuellen Form des Körperhohlraumpfads (41) erzeugt.

7. Biopsieunterstützungssystem (1B) gemäß Anspruch 1 oder 2, bei dem das Biopsiebehandlungsinstrument (51) einen Positionssensor (52) in der Nähe eines distalen Endabschnitts des Biopsiebehandlungsinstruments (51) umfasst, der Positionserfassungsabschnitt eine dreidimensionale Position des Positionssensors (52) des Biopsiebehandlungsinstruments (51) erfasst und

der Bildanzeigesteuerabschnitt (29) die dreidimensionale Position des Positionssensors (52) des Biopsiebehandlungsinstruments (21) einer entsprechenden Position auf dem Bild einer virtuellen Form überlagert anzeigt.

8. Biopsieunterstützungssystem (1B) gemäß Anspruch 7, das ferner einen Speicherabschnitt (33) umfasst, der eine Positionsinformation von beiden Endpositionen des Zielgewebes, die von dem Positionserfassungsabschnitt (8) erfasst werden, und eine Positionsinformation des Positionssensors (52) des Biopsiebehandlungsinstruments (51) speichert.

**Revendications**

1. Système de support de biopsie (1B) comprenant :

une section (16) de génération d'image de forme virtuelle qui génère une image de forme virtuelle d'un trajet (41) de cavité de corps à partir de données d'image dans une région en trois dimensions par rapport à un sujet ; un endoscope (18) muni d'une partie (19) d'insertion d'endoscope insérée dans le trajet (41) de cavité de corps et d'un canal (19a) qui permet à un instrument de traitement de biopsie (51) de passer à travers ; une sonde à ultrasons (2) insérée dans le canal (19a) incluant un transducteur à ultrasons (13) et un capteur de position (14) qui détecte une position en trois dimensions prévu au niveau d'une partie d'extrémité distale de celle-ci ; et une section (6) de génération d'image ultrasonore qui génère une image tomographique ultrasonore en utilisant le transducteur à ultrasons (13), **caractérisé en ce que** le système de support de biopsie (1B) comprend en outre une section (15) de détection de position qui détecte une position en trois dimensions du capteur de position (14), lorsque les deux positions d'extrémité d'un tissu cible devant être soumis à une biopsie sont détectées sur l'image tomographique ultrasonore ; une section (31) de commande de détection de position de tumeur qui détecte une position limite d'une tumeur en tant que tissu cible en utilisant la position en trois dimensions du capteur de position (14) détectée par la section (15) de détection de position, dans lequel la section (31) de commande de détection de position de tumeur est connectée à un commutateur (A34) de détection de tumeur qui reçoit en entrée un signal de détection de tumeur, des informations indiquant que la partie d'extrémité distale de la sonde à ultrasons (2) est déplacée le long d'une direction prédéterminée et qu'une extrémité de la tumeur est détectée, et un commutateur (B35) de détection de tumeur qui ne reçoit aucun signal de détection de tumeur en entrée, le commutateur (A34) de détection de tumeur et le commutateur (B35) de détection de tumeur étant actionnés par un opérateur ; et une section (29) de commande d'affichage d'image qui affiche une plage de biopsie dans laquelle la biopsie est réalisée sur le tissu cible en utilisant l'instrument de traitement de biopsie (51), superposée sur une position correspondant à la plage de biopsie sur l'image de forme virtuelle, sur la base de la position en trois dimensions correspondant aux deux positions d'extrémité détectées par la section (15) de détection de position, dans lequel la section (15) de détection de position détecte les positions en trois dimensions des deux positions d'extrémité du tissu cible le long de la direction de déplace-ment ($D_L$) du trajet (41) de cavité de corps, les deux positions d'extrémité étant détectées lorsque la partie d'extrémité distale de la sonde à ultrasons (2) est déplacée le long de la direction de déplacement ($D_L$), et détecte le centre ou le centre de gravité du tissu cible détecté le long de la direction de la largeur ($D_T$) orthogonale à la direction de déplacement ($D_L$) sur l'image tomographique ultrasonore, et la section (29) de commande d'affichage d'image affiche en outre la plage à l'intérieur des deux positions d'extrémité d'un tissu cible le long de la direction de déplacement ($D_L$) en tant que plage de biopsie superposée sur une position correspondant à la plage de biopsie sur l'image de forme virtuelle, et affiche le centre ou le centre de gravité du tissu cible le long de la direction de la largeur (d) superposé sur la position correspondant au centre ou au centre de gravité sur l'image de forme virtuelle.

2. Système de support de biopsie (1B) comprenant :

une section (16) de génération d'image de forme virtuelle qui génère une image de forme virtuelle d'un trajet (41) de cavité de corps à partir de données d'image dans une région en trois dimensions par rapport à un sujet ; un endoscope (18) muni d'une partie (19) d'insertion d'endoscope insérée dans le trajet (41) de cavité de corps et d'un canal (19a) qui permet à un instrument de traitement de biopsie (51) de passer à travers ; une sonde à ultrasons (2) insérée dans le canal (19a) incluant un transducteur à ultrasons (13) et un capteur de position (14) qui détecte une position en trois dimensions prévu au niveau d'une partie d'extrémité distale

EP 2 430 979 B1

de celle-ci ; et

une section (6) de génération d'image ultrasonore qui génère une image tomographique ultrasonore en utilisant le transducteur à ultrasons (13),

**caractérisé en ce que** le système de support de biopsie (1B) comprend en outre

une section (15) de détection de position qui détecte une position en trois dimensions du capteur de position (14), lorsque les deux positions d'extrémité d'un tissu cible devant être soumis à une biopsie sont détectées sur l'image tomographique ultrasonore ;

une section (31) de commande de détection de position de tumeur qui détecte une position limite d'une tumeur en tant que tissu cible en utilisant la position en trois dimensions du capteur de position (14) détectée par la section (15) de détection de position, dans lequel la section (31) de commande de détection de position de tumeur génère le signal de détection de tumeur lorsque, par l'intermédiaire du traitement d'image, l'image tomographique ultrasonore d'une tumeur en tant que tissu cible commence à être détectée, et ne génère aucun signal de détection de tumeur lorsque l'image tomographique ultrasonore cesse d'être détectée ; et

une section (29) de commande d'affichage d'image qui affiche une plage de biopsie dans laquelle la biopsie est réalisée sur le tissu cible en utilisant l'instrument de traitement de biopsie (51), superposée sur une position correspondant à la plage de biopsie sur l'image de forme virtuelle, sur la base de la position en trois dimensions correspondant aux deux positions d'extrémité détectées par la section (15) de détection de position, dans lequel la section (15) de détection de position détecte les positions en trois dimensions des deux positions d'extrémité du tissu cible le long de la direction de déplacement ($D_L$) du trajet (41) de cavité de corps, les deux positions d'extrémité étant détectées lorsque la partie d'extrémité distale de la sonde à ultrasons (2) est déplacée le long de la direction de déplacement ($D_L$), et détecte le centre ou le centre de gravité du tissu cible détecté le long de la direction de la largeur ($D_T$) orthogonale à la direction de déplacement ($D_L$) sur l'image tomographique ultrasonore, et

la section (29) de commande d'affichage d'image affiche en outre la plage à l'intérieur des deux positions d'extrémité d'un tissu cible le long de la direction de déplacement ($D_L$) en tant que plage de biopsie superposée sur une position correspondant à la plage de biopsie sur l'image de forme virtuelle, et affiche le centre ou le centre de gravité du tissu cible le long de la direction de la largeur ($D_T$) superposé sur la position correspondant au centre ou au centre de gravité sur l'image de forme virtuelle.

3. Système de support de biopsie (1B) selon la revendication 1 ou 2, dans lequel la section de détection de position détecte une position en trois dimensions d'une position de référence établie sur une face d'extrémité distale de l'endoscope (18) ou une périphérie de celui-ci, et

la section (29) de commande d'affichage d'image affiche la position de référence superposée sur une position sur l'image de forme virtuelle correspondant à la position de référence.

4. Système de support de biopsie (1B) selon la revendication 1 ou 2, dans lequel la section (29) de commande d'affichage d'image affiche, en supposant une plage établie à l'intérieur des deux positions d'extrémité sur la base des deux positions d'extrémité en tant que plage de biopsie, la plage de biopsie superposée sur une position correspondant à la plage de biopsie sur l'image de forme virtuelle.

5. Système de support de biopsie (1B) selon la revendication 1 ou 2, dans lequel la section de détection de position détecte une position en trois dimensions d'un centre de gravité ou une position centrale du tissu cible sur l'image tomographique ultrasonore, et

la section (29) de commande d'affichage d'image affiche le centre de gravité ou une position centrale superposée sur une position correspondant au centre de gravité ou à une position centrale sur l'image de forme virtuelle.

6. Système de support de biopsie (1B) selon la revendication 1 ou 2, dans lequel la section (16) de génération d'image de forme virtuelle génère une image de forme virtuelle de l'un quelconque parmi les bronches, le tract alimentaire supérieur et le tract alimentaire inférieur en tant qu'image de forme virtuelle du trajet (41) de cavité de corps.

7. Système de support de biopsie (1B) selon la revendication 1 ou 2, dans lequel l'instrument de traitement de biopsie (51) comprend un capteur de position (52) près d'une partie d'extrémité distale de l'instrument de traitement de biopsie (51),

la section de détection de position détecte une position en trois dimensions du capteur de position (52) de l'instrument de traitement de biopsie (51), et

la section (29) de commande d'affichage d'image affiche la position en trois dimensions du capteur de position (52) d'un instrument de traitement de biopsie (51) superposée sur une position correspondante sur l'image de forme virtuelle.

19

8. Système de support de biopsie (1B) selon la revendication 7, comprenant en outre une section de mémoire (33) qui mémorise des informations de position des deux positions d'extrémité du tissu cible détectées par la section de détection de position (8) et des informations de position du capteur de position (52) de l'instrument de traitement de biopsie (51).

# FIG.1

# FIG.2

# FIG.3

```
                    ( START )
                        │
                        ▼
┌──────────────────────────────────────────────────┐
│        GENERATE AND DISPLAY VIRTUAL IMAGE          │──S1
└──────────────────────────────────────────────────┘
                        │
                        ▼
┌──────────────────────────────────────────────────┐
│  INSERT ENDOSCOPE INTO BRONCHUS WITH REFERENCE TO  │
│   VIRTUAL IMAGE AND INSERT DISTAL END PORTION UP TO │──S2
│               VICINITY OF TARGET                    │
└──────────────────────────────────────────────────┘
                        │
                        ▼
┌──────────────────────────────────────────────────┐
│ INSERT ULTRASOUND PROBE INTO CHANNEL OF ENDOSCOPE  │──S3
│ AND CAUSE PROBE TO PROTRUDE FROM CHANNEL DISTAL END │
└──────────────────────────────────────────────────┘
                        │
                        ▼
┌──────────────────────────────────────────────────┐
│      BIOPSY SUPPORT SYSTEM STARTS DISPLAY OF ULTRASOUND │
│ TOMOGRAPHIC IMAGE AND DETECTION OF THREE-DIMENSIONAL │──S4
│  POSITION AND INSERTS ULTRASOUND PROBE SO AS TO MOVE │
│              CLOSER TO TARGET TISSUE                 │
└──────────────────────────────────────────────────┘
                        │
                        ▼
┌──────────────────────────────────────────────────┐
│     CHECK POSITION OF TARGET TISSUE ON ULTRASOUND   │──S5
│ TOMOGRAPHIC IMAGE AND INPUT TUMOR DETECTION SWITCH  │
└──────────────────────────────────────────────────┘
                        │
                        ▼
┌──────────────────────────────────────────────────┐
│ DETERMINE POSITION OF GUIDE TUBE BASED ON INFORMATION │
│  OF APPROPRIATE RANGE OR THE LIKE AND INFORMATION OF │──S6
│   POSITION OF PROBE DISTAL END AND THREE-DIMENSIONAL │
│            POSITION DISPLAYED ON MONITOR             │
└──────────────────────────────────────────────────┘
                        │
                        ▼
┌──────────────────────────────────────────────────┐
│ LEAVE GUIDE TUBE INSIDE CHANNEL, PULL OUT ULTRASOUND │──S7
│         PROBE AND INSERT BIOPSY DEVICE              │
└──────────────────────────────────────────────────┘
                        │
                        ▼
┌──────────────────────────────────────────────────┐
│ CAUSE BIOPSY DEVICE TO PROTRUDE FROM GUIDE TUBE AND │──S8
│             EXTRACT TARGET TISSUE                   │
└──────────────────────────────────────────────────┘
                        │
                        ▼
┌──────────────────────────────────────────────────┐
│         PULL OUT ENDOSCOPE FROM BRONCHUS            │──S9
└──────────────────────────────────────────────────┘
                        │
                        ▼
                    ( END )
```

# FIG.4

START

ACQUIRE CT IMAGE DATA —S11

EXTRACT BRONCHUS —S12

GENERATE VIRTUAL SHAPE IMAGE OF BRONCHUS —S13

GENERATE VBS IMAGE (VIRTUAL ENDOSCOPE IMAGE) —S14

DISPLAY VIRTUAL IMAGE OF BRONCHUS ON MONITOR —S15

END

# FIG.5

# FIG.6

# FIG.7

# FIG.8

```
                    ( START )
                        │
                        ▼                                    ╱S21
┌──────────────────────────────────────────────────────┐
│   ACQUIRE POSITION INFORMATION OF POSITION SENSOR      │◄──┐
└──────────────────────────────────────────────────────┘   │S22
                        │                                   │
                        ▼                            ╱S22   │
┌──────────────────────────────────────────────────────┐   │
│  DETECT THREE-DIMENSIONAL POSITION OF PROBE DISTAL END PORTION │ │
└──────────────────────────────────────────────────────┘   │
                        │                            ╱S23   │
                        ▼                                   │
┌──────────────────────────────────────────────────────┐   │
│ DISPLAY ULTRASOUND TOMOGRAPHIC IMAGE BY ULTRASOUND TRANSDUCER │ │
└──────────────────────────────────────────────────────┘   │
          S24                │                              │
            ◄─────────── IS TUMOR DETECTION SIGNAL INPUTTED? ───────►  N ──┘
                        │ Y
                        ▼                                    ╱S25
┌──────────────────────────────────────────────────────┐
│ RECORD THREE-DIMENSIONAL POSITION OF PROBE DISTAL END PORTION (TOP END POSITION A) │
└──────────────────────────────────────────────────────┘
                        │                            ╱S26
                        ▼
┌──────────────────────────────────────────────────────┐
│   ACQUIRE POSITION INFORMATION OF POSITION SENSOR      │◄──┐
└──────────────────────────────────────────────────────┘   │S27
                        │                            ╱S27   │
                        ▼                                   │
┌──────────────────────────────────────────────────────┐   │
│  DETECT THREE-DIMENSIONAL POSITION OF PROBE DISTAL END PORTION │ │
└──────────────────────────────────────────────────────┘   │
                        │                            ╱S28   │
                        ▼                                   │
┌──────────────────────────────────────────────────────┐   │
│ DISPLAY ULTRASOUND TOMOGRAPHIC IMAGE BY ULTRASOUND TRANSDUCER │ │
└──────────────────────────────────────────────────────┘   │
          S29                │                              │
            ◄─────────── IS NO TUMOR SIGNAL INPUTTED? ───────►  N ──────┘
                        │ Y
                        ▼                                    ╱S30
┌──────────────────────────────────────────────────────┐
│ RECORD THREE-DIMENSIONAL POSITION OF PROBE DISTAL END PORTION (BOTTOM END POSITION B) │
└──────────────────────────────────────────────────────┘
                        │                            ╱S31
                        ▼
┌──────────────────────────────────────────────────────┐
│ INPUT WIDTH DIRECTION Dt IN WHICH TUMOR EXISTS AND RECORD WIDTH DIRECTION Dt │
└──────────────────────────────────────────────────────┘
                        │                            ╱S32
                        ▼
┌──────────────────────────────────────────────────────┐
│        READ APPROPRIATE RANGE PARAMETER OF BIOPSY      │
└──────────────────────────────────────────────────────┘
                        │                            ╱S33
                        ▼
┌──────────────────────────────────────────────────────┐
│              CALCULATE APPROPRIATE RANGE K             │
└──────────────────────────────────────────────────────┘
                        │                            ╱S34
                        ▼
┌──────────────────────────────────────────────────────┐
│ DISPLAY APPROPRIATE RANGE K AND WIDTH DIRECTION Dt OR THE LIKE SUPERIMPOSED ON VIRTUAL IMAGE │
└──────────────────────────────────────────────────────┘
                        │                            ╱S35
                        ▼
┌──────────────────────────────────────────────────────┐
│   ACQUIRE POSITION INFORMATION OF POSITION SENSOR      │◄──┐
└──────────────────────────────────────────────────────┘   │S36
                        │                            ╱S36   │
                        ▼                                   │
┌──────────────────────────────────────────────────────┐   │
│ DETECT THREE-DIMENSIONAL POSITION OF PROBE DISTAL END PORTION AND DISPLAY │ │
│              SUPERIMPOSED ON VIRTUAL IMAGE              │   │
└──────────────────────────────────────────────────────┘   │
                        │                            ╱S37   │
                        ▼                                   │
┌──────────────────────────────────────────────────────┐   │
│ SET PROBE DISTAL END PORTION ON DISTAL END FACE OF ENDOSCOPE AND DISPLAY │ │
│  POSITION OF DISTAL END FACE SUPERIMPOSED ON VIRTUAL IMAGE │ │
└──────────────────────────────────────────────────────┘   │
          S38                │                              │
            ◄─────────── APPROPRIATE RANGE RESET? ───────►  N ─────────┘
                        │ Y
                        ▼
                    ( END )
```

# FIG.9

# FIG.10

# FIG.11

TUMOR IS NOT VISIBLE

TUMOR IS VISIBLE

# FIG.12

ENLARGED VIEW

# FIG.13

# FIG.14

```
              ┌─────────────┐
              │    START    │
              └──────┬──────┘
                     ▼                                              ╱S21
┌──────────────────────────────────────────────────────────────┐
│        ACQUIRE POSITION INFORMATION OF POSITION SENSOR         │◄──┐
└──────────────────────────┬─────────────────────────────────────┘   │
                           ▼                                    ╱S22   │
┌──────────────────────────────────────────────────────────────┐    │
│    DETECT THREE-DIMENSIONAL POSITION OF PROBE DISTAL END PORTION │    │
└──────────────────────────┬─────────────────────────────────────┘   │
                           ▼                                    ╱S23   │
┌──────────────────────────────────────────────────────────────┐    │
│   DISPLAY ULTRASOUND TOMOGRAPHIC IMAGE BY ULTRASOUND TRANSDUCER │    │
└──────────────────────────┬─────────────────────────────────────┘   │
         S24'               ▼                                          │
         ◄────────── IS TUMOR DETECTION SIGNAL DETECTED? ──────► N ───┤
                           ▼ Y                                  ╱S25   │
┌──────────────────────────────────────────────────────────────┐    │
│ RECORD THREE-DIMENSIONAL POSITION OF PROBE DISTAL END PORTION (TOP END POSITION A) │
└──────────────────────────┬─────────────────────────────────────┘
                           ▼                                    ╱S26
┌──────────────────────────────────────────────────────────────┐
│        ACQUIRE POSITION INFORMATION OF POSITION SENSOR         │◄──┐
└──────────────────────────┬─────────────────────────────────────┘   │
                           ▼                                    ╱S27   │
┌──────────────────────────────────────────────────────────────┐    │
│    DETECT THREE-DIMENSIONAL POSITION OF PROBE DISTAL END PORTION │    │
└──────────────────────────┬─────────────────────────────────────┘   │
                           ▼                                    ╱S28   │
┌──────────────────────────────────────────────────────────────┐    │
│   DISPLAY ULTRASOUND TOMOGRAPHIC IMAGE BY ULTRASOUND TRANSDUCER │    │
└──────────────────────────┬─────────────────────────────────────┘   │
         S29'               ▼                                          │
         ◄────────────── IS NO TUMOR SIGNAL DETECTED? ────────► N ────┤
                           ▼ Y                                  ╱S30
┌──────────────────────────────────────────────────────────────┐
│ RECORD THREE-DIMENSIONAL POSITION OF PROBE DISTAL END PORTION (BOTTOM END POSITION B) │
└──────────────────────────┬─────────────────────────────────────┘
                           ▼                                    ╱S31
┌──────────────────────────────────────────────────────────────┐
│ INPUT WIDTH DIRECTION Dт IN WHICH TUMOR EXISTS AND RECORD WIDTH DIRECTION Dт │
└──────────────────────────┬─────────────────────────────────────┘
                           ▼                                    ╱S32
┌──────────────────────────────────────────────────────────────┐
│          READ APPROPRIATE RANGE PARAMETER OF BIOPSY           │
└──────────────────────────┬─────────────────────────────────────┘
                           ▼                                    ╱S33
┌──────────────────────────────────────────────────────────────┐
│               CALCULATE APPROPRIATE RANGE K                   │
└──────────────────────────┬─────────────────────────────────────┘
                           ▼                                    ╱S34
┌──────────────────────────────────────────────────────────────┐
│ DISPLAY APPROPRIATE RANGE K AND WIDTH DIRECTION Dт OR THE LIKE SUPERIMPOSED ON VIRTUAL IMAGE │
└──────────────────────────┬─────────────────────────────────────┘
                           ▼                                    ╱S35
┌──────────────────────────────────────────────────────────────┐
│        ACQUIRE POSITION INFORMATION OF POSITION SENSOR         │◄──┐
└──────────────────────────┬─────────────────────────────────────┘   │
                           ▼                                    ╱S36   │
┌──────────────────────────────────────────────────────────────┐    │
│ DETECT THREE-DIMENSIONAL POSITION OF PROBE DISTAL END PORTION AND DISPLAY │
│              SUPERIMPOSED ON VIRTUAL IMAGE                     │    │
└──────────────────────────┬─────────────────────────────────────┘   │
                           ▼                                    ╱S37   │
┌──────────────────────────────────────────────────────────────┐    │
│ SET PROBE DISTAL END PORTION ON DISTAL END FACE OF ENDOSCOPE AND DISPLAY │
│   POSITION OF DISTAL END FACE SUPERIMPOSED ON VIRTUAL IMAGE   │    │
└──────────────────────────┬─────────────────────────────────────┘   │
        S38                ▼                                          │
        ◄────────────── APPROPRIATE RANGE RESET? ──────────► N ──────┘
                           ▼ Y
              ┌─────────────┐
              │     END     │
              └─────────────┘
```

# FIG.15

TOMOGRAPHIC
IMAGE DATA

ULTRASOUND
TRANSDUCER 13 AT
TOP END POSITION A

ULTRASOUND
TRANSDUCER 13 AT
END POSITION f

ULTRASOUND
TRANSDUCER 13 AT
END POSITION e

(f)

(e)

$D_T$

$D_L$

42

APPROPRIATE
RANGE J

F

E

# FIG.16

```
          ( START )
              │
              ▼
┌─────────────────────────────────────────────┐
│ PERFORM POSITION DETECTION WHILE MOVING PROBE │
│ DISTAL END PORTION OF ULTRASOUND PROBE FROM TOP │──S41
│ END POSITION A TO BOTTOM END POSITION AND RECORD │
│          POSITION INFORMATION                 │
└─────────────────────────────────────────────┘
              │
              ▼
┌─────────────────────────────────────────────┐
│ RECORD TOMOGRAPHIC IMAGE DATA AT EACH POSITION IN │──S42
│    CONJUNCTION WITH POSITION DETECTION        │
└─────────────────────────────────────────────┘
              │
              ▼
┌─────────────────────────────────────────────┐
│ DETECT BOTH END POSITIONS e AND f ALONG WIDTH │──S43
│    DIRECTION $D_T$ ON TOMOGRAPHIC IMAGE       │
└─────────────────────────────────────────────┘
              │
              ▼
┌─────────────────────────────────────────────┐
│ CALCULATE DISTANCES We AND Wf FROM ULTRASOUND │
│   TRANSDUCER TO BOTH END POSITIONS e AND f ON │──S44
│          TOMOGRAPHIC IMAGE                    │
└─────────────────────────────────────────────┘
              │
              ▼
┌─────────────────────────────────────────────┐
│ CALCULATE THREE-DIMENSIONAL POSITIONS OF BOTH END │
│ POSITIONS E AND F OF TUMOR FROM DISTANCES We AND Wf ON │──S45
│ TOMOGRAPHIC IMAGE USING THREE-DIMENSIONAL POSITION │
│    INFORMATION OF ULTRASOUND TRANSDUCER       │
└─────────────────────────────────────────────┘
              │
              ▼
┌─────────────────────────────────────────────┐
│ CALCULATE APPROPRIATE RANGE J IN WIDTH DIRECTION $D_T$ │──S46
│      FROM BOTH END POSITIONS E AND F          │
└─────────────────────────────────────────────┘
              │
              ▼
┌─────────────────────────────────────────────┐
│ DISPLAY APPROPRIATE RANGES K AND J OR THE LIKE │──S47
│      SUPERIMPOSED ON VIRTUAL IMAGE            │
└─────────────────────────────────────────────┘
              │
              ▼
          (  END  )
```

# FIG.17

# FIG.18

# FIG.19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004000499 A
- JP 4022192 B
- WO 2008111070 A1
- WO 2004042546 A1
- US 2005215854 A1